# EUROPEAN PATENT APPLICATION

(11) **EP 4 830 745 A1**
(43) Date of publication of application: **28.10.2026**
(21) Application number: 26173869.4
(22) Date of filing: 22.04.2026
(51) Int. Cl.: A61M 5/142, A61M 5/145, A61M 5/158

(54) **DEVICE FOR SUBCUTANEOUS DELIVERY OF A MEDICAMENT**

(30) Priority: 24.04.2025 IT 202500009201
(71) Applicant: Nuova Ompi S.r.l., 35017 Piombino Dese, (PD) (IT)
(72) Inventor: RIVA, Christian, 20092 Cinisello Balsamo (MI) (IT); DEGAN, Paolo, 24020 Ranica (BG) (IT); GIACOMOZZI, Andrea, 20131 Milano (MI) (IT); NICOLI, Ilaria, 43123 Parma (PR) (IT); DE CHIRICO BACER, Luca, 20129 Milano (MI) (IT); VALSECCHI, Luca, 20126 Milano (MI) (IT)
(74) Representative: Porta & Consulenti Associati S.p.A.

(57) **Abstract**

A device (1) for subcutaneous delivery of a medicament, comprising a rotor (17) selectively controllable in rotation in a first rotation direction (R1) and in a second rotation direction (R2) opposite the first rotation direction (R1). The device (1) further comprises a motion transmission mechanism (70) coupled to the rotor (17) and configured to transfer motion from the rotor (17) to a first motion transmission member (80) when the rotor (17) rotates in said first rotation direction (R1) and from the rotor (17) to a second motion transmission member (85) when the rotor (17) rotates in said second rotation direction (R2). The first motion transmission member (80) and the second motion transmission member (85) are arranged on opposite sides with respect to the motion transmission mechanism (70). The latter comprises a service shaft (74) selectively movable towards the first motion transmission mechanism (80) and the second motion transmission mechanism (85) so as to be coupled to the first motion transmission member (80) and decoupled from the second motion transmission member (85) when the rotor (17) rotates in said first rotation direction (R1) and vice versa when the rotor (17) rotates in said second rotation direction (R2).

## Description

The present invention relates to a device for subcutaneous delivery of a medicament.

In particular, the device of the present invention falls into the category of "infusion pumps" as defined by the FDA (US Food and Drug Administration) and relates to, more particularly, a device of the wearable type, i.e. configured to be applied on the body of a patient in order to allow the subcutaneous administration of a predetermined dose of a medicament. The application of the device on the body of the patient is carried out by a user, for example a doctor or a nurse or the patient himself/herself.

In all the wearable delivery devices the medicament is initially contained in a cartridge housed inside the device and is transferred to the body of the patient through a fluidic path.

Hereinafter the expression "fluidic path" is used to indicate any element or assembly of elements configured to be connected, at a first end thereof, to the cartridge housed inside the device and comprising, at an end thereof opposed to the aforementioned first end, an injection needle configured to be inserted into the body of the patient to allow the flow of the medicament from the cartridge to the body of the patient.

Typically, the cartridge comprises a cylindrical container, made of plastic or glass material, a plunger slidable inside the container to push the medicament out of the container and a pierceable septum that guarantees the sterility of the container until the beginning of the administration of the therapy.

Typically, the fluidic path comprises a piercing needle configured to pierce the pierceable septum of the cartridge and a flexible tube that puts the piercing needle in fluid communication with the injection needle.

The container of the cartridge is kept closed by the pierceable septum until the administration of the medicament is activated. Following this activation, the piercing needle pierces the pierceable septum and opens the fluidic path, allowing the medicament to reach the patient by initially passing through the piercing needle, then through the flexible tube and finally through the injection needle. The movement of the medicament occurs by the thrust exerted on it by the plunger inside the container.

An example of a delivery device of the type discussed above is described in WO 2020/128821A1.

Such a device comprises a rotor rotatable about an axis of rotation in a first rotation direction and in a second rotation direction opposite the first rotation direction.

The rotor is coupled to a control shaft that is connected, at its opposite ends, to two different motion transmission members: a member configured to transmit motion to a fluid injection device and a member configured to transmit motion to a cannula extraction device.

The connection between the control shaft and each of the aforementioned motion transmission members takes place via a respective unidirectional transmission mechanism comprising an asymmetrical cam provided with a deformable element.

The asymmetrical cams are mounted in such a way that, when the control shaft is rotated in one rotation direction, only one of the two unidirectional transmission mechanisms is active and, consequently, motion is transferred from the rotor via the aforementioned control shaft to only one of the aforementioned motion transmission members. Instead, when the control shaft is rotated in the other rotation direction, only the other unidirectional transmission mechanism is active and, consequently, motion is transferred from the rotor via the aforementioned control shaft only to the other of the aforementioned motion transmission members.

In particular, the activation of the unidirectional transmission mechanism is achieved by the thrust exerted by a tooth of a toothing provided on the control shaft on a tooth provided on the deformable element of the asymmetrical cam.

When the unidirectional transmission mechanism is inactive, the teeth of the control shaft toothing go one after the other into abutment on the tooth of the deformable element of the asymmetrical cam, each time causing an elastic deformation of the deformable element, which allows the tooth of the deformable element to ride over the teeth of the control shaft toothing.

The Applicant found that a unidirectional transmission mechanism of the type described above, when inactive, generates a noise that is repeated whenever the tooth of the deformable element of the respective asymmetrical cam rides over a tooth of the control shaft.

Investigations carried out by the Applicant in relation to the aforementioned noise revealed that although for some users this noise is advantageous, as it is an indication that the delivery device is operating, for other users the aforementioned noise is a source of annoyance and/or is interpreted as an indication of malfunctioning of the delivery device.

The technical problem solved by the present invention is to provide a device for subcutaneous delivery of a medicament that does not make such a noise during operation.

The Applicant observed that the aforementioned noise is a consequence of the fact that the control shaft is engaged with both the asymmetrical cams both when the control shaft turns in one rotation direction to transfer motion from the rotor to one of the aforementioned motion transmission members and when the control shaft turns in the opposite direction to transfer motion from the rotor to the other of the aforementioned motion transmission members. Thus, there is always a unidirectional transmission mechanism at which this noise is generated.

Accordingly, the Applicant thought to provide a motion transmission mechanism such that when the shaft that receives motion from the rotor turns in one rotation direction it is connected to only one of the aforementioned motion transmission members and when the aforementioned shaft turns in the opposite direction the motion transmission mechanism is connected only to the other of the aforementioned motion transmission members.

The present invention therefore relates to a device for subcutaneous delivery of a medicament, comprising:
- a rotor selectively controllable in rotation in a first rotation direction and a second rotation direction opposite the first rotation direction;
- a motion transmission mechanism coupled to the rotor and configured to transfer motion from the rotor to a first motion transmission member when the rotor rotates in said first rotation direction and from the rotor to a second motion transmission member when the rotor rotates in said second rotation direction, wherein the first motion transmission member and the second motion transmission member are arranged on opposite sides with respect to the motion transmission mechanism;
characterised in that the motion transmission mechanism comprises a service shaft selectively movable towards the first motion transmission member and the second motion transmission member so as to:
- be coupled to the first motion transmission member and decoupled from the second motion transmission member when the rotor rotates in said first rotation direction;
- be coupled to the second motion transmission member and decoupled from the first motion transmission member when the rotor rotates in said second rotation direction.

In the device of the invention therefore a selective coupling with only one of the two motion transmission members is provided for, depending on the rotation direction of the service shaft. Such a selective coupling is achieved by the movement (in particular, the translation) of the service shaft towards one of the aforementioned motion transmission members when the service shaft rotates in a first rotation direction and towards the other of the aforementioned motion transmission members when the service shaft rotates in the opposite direction, resulting in the selective decoupling of the service shaft with the motion transmission member other than the one which each time is coupled with the service shaft.

Advantageously, the aforementioned selective coupling makes it possible to prevent a noise such as the one discussed above from being generated at the motion transmission member that is decoupled from the service shaft and, therefore, inactive. In fact, this motion transmission member is not subject to any stress from the service shaft and, therefore, cannot be a source of noise.

Moreover, thanks to such a selective decoupling, the two motion transmission members are subject to much less wear than they would be if both were always engaged with the service shaft.

Preferred features of the device of the invention are described hereinbelow, wherein each of these features can be provided individually or in combination with the others.

Preferably, the motion transmission mechanism also comprises a worm screw solidly associated with the rotor.

Preferably, the motion transmission mechanism comprises a toothed wheel solidly associated with the service shaft.

Preferably, the toothed wheel is engaged with the worm screw. The engagement between the worm screw and toothed wheel allows the transfer of motion from the rotor to the service shaft.

Preferably, the worm screw comprises a first helical toothing.

Throughout this description and in subsequent claims, the term "axial", when referring to any element or member intended to rotate about its own axis of rotation, is used to indicate a direction parallel to or coinciding with said axis of rotation, while the term "circumferential", when also referring to any element or member intended to rotate about its own axis of rotation, is used to indicate a direction that revolves around said axis of rotation.

Preferably, the toothed wheel comprises a second helical toothing conjugated to the first helical toothing. In this way, a gear is created that is capable of generating an axial force component in the service shaft to achieve the selective movement of the service shaft towards the two motion transmission members.

In this description and in the subsequent claims, when reference is made to certain angle values, absolute values are meant, i.e. both positive and negative values with respect to a reference plane or direction, unless otherwise specified.

Furthermore, when reference is made to any range of values between a minimum value and a maximum value, the aforementioned minimum and maximum values are understood to be included in the said range, unless expressly provided otherwise.

Furthermore, all the ranges include any combination of the maximum and minimum values described and include any intermediate range, even if not specifically described.

Even if not expressly indicated, any numerical value is understood to be preceded by the term "about" to also indicate any numerical value that deviates slightly from the one described, e.g. to take account of dimensional tolerances typical of the relevant field.

The Applicant paid special attention to appropriately size the helical toothing of the toothed wheel (i.e. the aforementioned second helical toothing) and the helical toothing of the worm screw (i.e. the aforementioned first helical toothing).

In particular, the Applicant made a number of considerations that led to appropriately select a range of values for the angle of inclination of the helical toothing of the toothed wheel and, consequently, of the helical toothing of the worm screw.

The Applicant observed that the angle of inclination of the helical toothing of the service shaft is inversely proportional to the motion transmission efficiency between worm screw and service shaft and directly proportional to the axial force component on the service shaft, i.e. low angles of inclination result in high motion transmission efficiency while high angles of inclination result in high force axial components.

The Applicant also noted that the angle of inclination of the helical toothing of the service shaft must be sufficiently high to ensure that the service shaft receives an axial force component capable of overcoming the frictional force that inevitably opposes the axial movement of the service shaft.

The Applicant also noted that the sizing of the aforementioned helical toothings must necessarily be carried out taking in due consideration the space inside the delivery device intended to house the motion transmission mechanism. Smaller angles of inclination can be used with toothed wheels and worm screws having larger diameters, and vice versa.

Taking into consideration all the aspects discussed above, the Applicant has found that angles of inclination of the second helical toothing, with respect to a plane containing the axis of rotation of the toothed wheel, preferably comprised between 5° and 30°, are suitable to guarantee an axial force component sufficient to overcome the aforementioned friction force and to minimise the loss of efficiency in motion transmission.

Instead, with regard to the helical toothing of the worm screw, the Applicant observed that the angle of inclination of this helical toothing depends on the number of teeth of the helical toothing of the toothed wheel and on the distance between the axis of rotation of the service shaft and the worm screw. The Applicant has found that for a single-thread worm screw, the first helical toothing must be sized in such a way that, at each revolution of the worm screw, the next tooth of the second helical toothing is engaged.

Preferably, the toothed wheel has a number of teeth comprised between 12 and 25, for example equal to 15.

Preferably, the toothed wheel has a modulus comprised between 0.3 mm and 1 mm, for example equal to 0.5 mm. The "modulus" of a toothed wheel is understood to be the ratio of the primitive diameter of the toothing of the toothed wheel to the number of teeth of the toothing of the toothed wheel, and the "primitive diameter" of a toothing is understood to be the diameter of the circumference at which the contact between the toothing and another toothing occurs.

Preferably, the second helical toothing has a primitive diameter comprised between 3.6 mm and 28.9 mm, for example equal to 7.6 mm.

Preferably, the first helical toothing has a nominal primitive diameter comprised between 0.5 mm and 11.4 mm, for example equal to 3.6 mm.

Preferably, the service shaft comprises a first end portion configured to be rotationally coupled to the first motion transmission member.

Preferably, the service shaft also comprises a second end portion opposite the first end portion and configured to be rotationally coupled to the second motion transmission member.

Preferably, the first motion transmission member comprises a first axial hole configured to house said first end portion when said first end portion is rotationally coupled to the first motion transmission member.

Preferably, said first axial hole is a blind hole.

Preferably, the second motion transmission member comprises a second axial hole configured to house said second end portion when said second end portion is rotationally coupled to the second motion transmission member.

Preferably, the second axial hole is a through hole.

The provision of the aforementioned first and second axial holes allows the axial footprint of the device to be contained.

Preferably, the first motion transmission member comprises a first abutment element.

Preferably, said first end portion comprises a first thrust element configured to cooperate in abutment with said first abutment element when said first end portion is rotationally coupled to the first motion transmission member.

Preferably, the second motion transmission member comprises a second abutment element.

Preferably, said second end portion comprises a second thrust element configured to cooperate in abutment with said second abutment element when said second end portion is rotationally coupled to the second motion transmission member.

Preferably, the motion transmission mechanism is housed within a pre-assembled unit.

Preferably, the first motion transmission member is supported by said pre-assembled unit.

Preferably, the second motion transmission member is supported by said pre-assembled unit.

The provision of the aforementioned pre-assembled unit ensures the correct mutual positioning of the motion transmission member, the first motion transmission member and the second motion transmission member, simplifying the device assembly operations.

In some of preferred embodiments thereof, the service shaft comprises a pair of annular grooves arranged between said first end portion and said second end portion.

Preferably, the pre-assembled unit comprises at least one elastically deformable coupling element arranged in proximity to said pair of annular grooves.

Preferably, said at least one coupling element comprises a free end portion housed in a first annular groove of said pair of annular grooves when the service shaft is rotationally coupled to the first motion transmission member.

Preferably, said free end portion is housed in a second annular groove of said pair of annular grooves when the service shaft is rotationally coupled to the second motion transmission member.

The Applicant has in fact observed that, due to the unavoidable play present between the service shaft and the motion transmission member coupled thereto, and/or due to the gravity and/or possible shocks and/or vibrations, each time the transmission of motion from the service shaft to either of the two motion transmission member ends (i.e. each time the rotor stops rotating in a predetermined rotation direction) a backward axial displacement of the service shaft and, consequently, a decoupling between the service shaft and the motion transmission member could occur. Such a decoupling is undesirable, especially when the device is provided to administer a pre-determined dose of the medicament in several successive deliveries. In this case, when the rotor is put again into rotation in order to carry out a subsequent delivery, during a first part of this rotation there would be a displacement of the service shaft towards the aforementioned motion transmission member without any delivery of the medicament, thus reducing the precision of the quantity of medicament administered. Having provided that, when the service shaft is coupled to a respective motion transmission member, the free end portion of the aforementioned at least one coupling element is housed in a respective annular groove provided on the service shaft, prevents any undesirable axial backward displacement of the service shaft in the situations discussed above.

Preferably, the transition of said at least one coupling element from said first annular groove to said second annular groove occurs thanks to the elastic deformation of said at least one coupling element.

Preferably, two coupling elements of the type described above are provided, wherein said coupling elements are arranged on opposing sides with respect to said service shaft so that the free end portion of each of them is housed in said first annular groove when the service shaft is rotationally coupled to the first motion transmission member and in said second annular groove when the service shaft is rotationally coupled to the second motion transmission member.

Preferably, the first motion transmission member is in axial abutment against a respective inner wall of said pre-assembled unit.

Preferably, the second motion transmission member is in axial abutment against a respective inner wall of said pre-assembled unit.

Preferably, the worm screw is in abutment against a further inner wall of said pre-assembled unit.

In this way, the two motion transmission members and the worm screw can only rotate inside the device when they are controlled in rotation by the rotor.

Preferably, said rotor is a driven rotor.

Preferably, said driven rotor is selectively controllable in rotation by a driving rotor.

The provision of two rotors makes it possible to make the device in two mutually couplable modules, one comprising the driving rotor and all the components necessary to activate the device and the other comprising the driven rotor and all the components necessary to deliver the medicament, the first module being reusable and the second module being disposable.

Preferably, the driven rotor is controlled in rotation by the driving rotor due to a magnetic coupling between the driven rotor and the driving rotor. In the case where the device is produced in two modules as discussed above, the provision of a magnetic coupling between the two rotors makes it possible to avoid that coupling members to the other module from protrude from each module.

Preferably, the driving rotor is selectively controllable in rotation in said first rotation direction and said second rotation direction by a motor.

Preferably, the driving rotor comprises at least one first permanent magnet having a first polarity and at least one second permanent magnet having a second polarity opposite the first polarity.

Preferably, the driven rotor also comprises at least one first permanent magnet having a first polarity and at least one second permanent magnet having a second polarity opposite the first polarity.

Preferably, the driving rotor is arranged above the driven rotor when the device is made in two modules and these modules are coupled together.

Preferably, the driving rotor comprises at least one pair of first permanent magnets having said first polarity and at least one pair of second permanent magnets having said second polarity.

Preferably, the driven rotor also comprises at least one pair of first permanent magnets having said first polarity and at least one pair of second permanent magnets having said second polarity.

Preferably, each magnet of said at least one pair of first permanent magnets of the driving rotor and of said at least one pair of second permanent magnets of the driving rotor is arranged above a respective area defined between a respective magnet of said at least one pair of first permanent magnets of the driven rotor and a respective magnet of said at least one pair of second permanent magnets of the driven rotor. The Applicant has found that this arrangement allows to achieve and maintain a stable equilibrium condition between the driving rotor and the driven rotor, preventing any slippage of one rotor with respect to the other or immediately restoring a stable equilibrium condition in the event that for some reasons (shocks, fall, etc.) such a slippage occurs.

Preferably, in each of said driving rotor and driven rotor the magnets of said at least one pair of first permanent magnets and of said at least one pair of second permanent magnets are circumferentially arranged one after the other.

Preferably, the magnets of said at least one pair of first permanent magnets are circumferentially interposed between the magnets of said at least one pair of second permanent magnets. The Applicant found that such an alternating arrangement allows the internal stresses in each rotor to be evenly distributed. This is due to the fact that each of the magnets in each of the two rotors is attracted by an adjacent magnet and repelled by another adjacent magnet with identical attraction/repulsion force.

Preferably, at least one of the driving rotor and the driven rotor comprises a ferromagnetic ring arranged on the opposite side with respect to the other of the driving rotor and the driven rotor. The provision of such a ring allows the magnetic field strength in the area between the rotors to be increased.

In a further aspect thereof, the present invention relates to a device for subcutaneous delivery of a medicament, comprising:
- a rotor selectively controllable in rotation in a first rotation direction and a second rotation direction opposite the first rotation direction;
- a motion transmission mechanism coupled to the rotor and configured to transfer motion from the rotor to a first motion transmission member when the rotor rotates in said first rotation direction and from the rotor to a second motion transmission member when the rotor rotates in said second rotation direction, wherein the first motion transmission member and the second motion transmission member are arranged on opposite sides with respect to the motion transmission mechanism;

wherein said rotor is a driven rotor selectively controllable in rotation by a driving rotor due to a magnetic coupling between the driven rotor and the driving rotor, the driving rotor being arranged above the driven rotor;
wherein the driving rotor is selectively controllable in rotation in said first rotation direction and said second rotation direction by a motor;
wherein the driving rotor and the driven rotor each comprise at least one pair of first permanent magnets having a first polarity and at least one pair of second permanent magnets having a second polarity opposite the first polarity, each magnet of said at least one pair of first permanent magnets of the driving rotor and said at least one pair of second permanent magnets of the driving rotor being arranged above a respective area defined between a respective magnet of said at least one pair of first permanent magnets of the driven rotor and a respective magnet of said at least one pair of second permanent magnets of the driven rotor.

In a further aspect thereof, the present invention relates to a device for subcutaneous delivery of a medicament, comprising:
- a rotor selectively controllable in rotation in a first rotation direction and a second rotation direction opposite the first rotation direction;
- a motion transmission mechanism coupled to the rotor and configured to transfer motion from the rotor to a first motion transmission member when the rotor rotates in said first rotation direction and from the rotor to a second motion transmission member when the rotor rotates in said second rotation direction, wherein the first motion transmission member and the second motion transmission member are arranged on opposite sides with respect to the motion transmission mechanism;

wherein said rotor is a driven rotor selectively controllable in rotation by a driving rotor due to a magnetic coupling between the driven rotor and the driving rotor, the driving rotor being arranged above the driven rotor;
wherein the driving rotor is selectively controllable in rotation in said first rotation direction and said second rotation direction by a motor;
wherein the driving rotor and the driven rotor each comprise at least one pair of first permanent magnets having a first polarity and at least one pair of second permanent magnets having a second polarity opposite the first polarity, each magnet of said at least one pair of first permanent magnets of the driving rotor and said at least one pair of second permanent magnets of the driving rotor being arranged above a respective area defined between a respective magnet of said at least one pair of first permanent magnets of the driven rotor and a respective magnet of said at least one pair of second permanent magnets of the driven rotor;
wherein in each of said driving rotor and driven rotor the magnets of said at least one pair of first permanent magnets and of said at least one pair of second permanent magnets are circumferentially arranged one after the other, the magnets of said at least one pair of first permanent magnets being circumferentially interposed between the magnets of said at least one pair of second permanent magnets.

In a further aspect thereof, the present invention relates to a device for subcutaneous delivery of a medicament, comprising:
- a rotor selectively controllable in rotation in a first rotation direction and a second rotation direction opposite the first rotation direction;
- a motion transmission mechanism coupled to the rotor and configured to transfer motion from the rotor to a first motion transmission member when the rotor rotates in said first rotation direction and from the rotor to a second motion transmission member when the rotor rotates in said second rotation direction, wherein the first motion transmission member and the second motion transmission member are arranged on opposite sides with respect to the motion transmission mechanism;

wherein said rotor is a driven rotor selectively controllable in rotation by a driving rotor due to a magnetic coupling between the driven rotor and the driving rotor;
wherein the driving rotor is selectively controllable in rotation in said first rotation direction and said second rotation direction by a motor;
wherein at least one of the driving rotor and the driven rotor comprises a ferromagnetic ring arranged on the opposite side with respect to the other between the driving rotor and the driven rotor.

Further characteristics and advantages of the present invention will become clearer from the following detailed description of the preferred embodiments thereof, made with reference to the appended drawings and provided by way of indicative and non-limiting example. In such drawings:
- Figure 1 is a perspective view from above of a device according to the present invention;
- Figure 2 is a perspective view from below of the device of Figure 1 ready to be applied on a patient's body (for clarity of illustration, the adhesive layer necessary to enable the device to be applied on the patient's body is not shown);
- Figure 3 is a perspective view from below of the device of Figure 1 in an operational configuration thereof prior to that of Figure 2;
- Figure 4 is an exploded perspective view of a delivery module of the device of Figure 1;
- Figure 5 is a perspective view of some components of the delivery module of Figure 4;
- Figure 6 is a perspective view of some components of Figure 5;
- Figure 7 is a perspective view of some components of the delivery module of Figure 4;
- Figure 8 is a perspective view of some components of Figure 5;
- Figure 9 is a perspective view of some components of Figure 5;
- Figure 10 is a sectional side view of the components of Figure 7;
- Figure 11 is a cross-sectional side view of some components of the delivery module of Figure 4;
- Figure 12 is a perspective view of a portion of the delivery module of Figure 4;
- Figures 13 and 14 are plan views of a portion of the delivery module of Figure 4, in two operational configurations;
- Figures 15 and 16 are cross-sectional views of another portion of the delivery module of Figure 4, in two operational configurations;
- Figures 17 and 18 are plan views of a portion of the delivery module of Figure 4 containing those of Figures 13-16, in two operational configurations;
- Figures 19 and 20 are perspective and plan views of the delivery module of Figure 4, in an operational configuration thereof;
- Figure 21 is a cross-sectional view of a portion of the delivery module of Figure 4 containing the one of Figures 15 and 16;
- Figures 22 and 23 are plan views of a different portion of the delivery module of Figure 4, in two operational configurations;
- Figure 24 is a plan view of a portion of the delivery module of Figure 4 similar to those of Figures 17 and 18, in a different operational configuration from the one of Figures 17 and 18;
- Figure 25 is a cross-sectional view of a portion of the delivery module of Figure 4 similar to the one of Figure 21, in a different operational configuration from the one of Figure 21;
- Figure 26 is a sectional view of a detail of the portion of Figure 25, in an operational configuration;
- Figure 27 is a perspective view of a component of the delivery module of Figure 4;
- Figure 28 is a plan view from below of the delivery module of Figure 4;
- Figure 29 is a cross-sectional view of the device of Figure 1;
- Figure 30 is a perspective view of an alternative embodiment of some components of the delivery module of Figure 4;
- Figure 31 is a cross-sectional side view of an alternative embodiment of the delivery module of Figure 4, comprising the components of Figure 30.

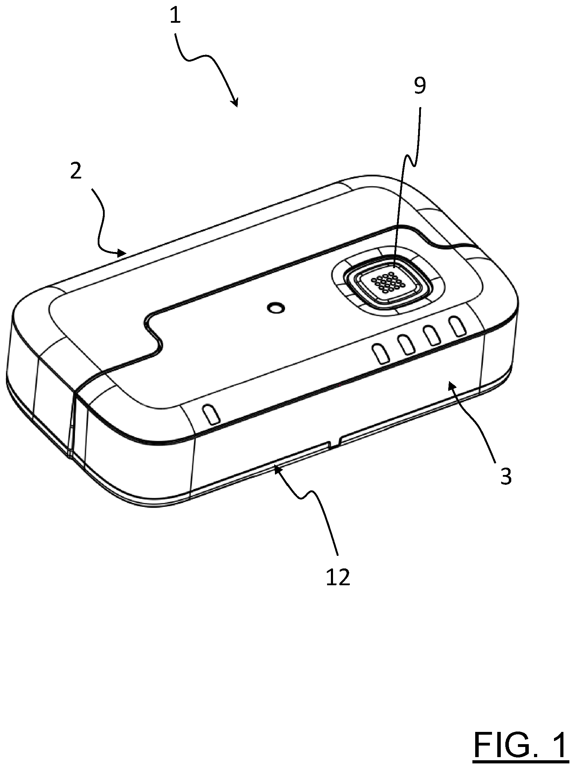

In Figure 1, a device of the wearable type for the subcutaneous delivery of a medicament in accordance with the present invention is indicate with numeral reference 1.

The device 1 comprises a delivery module 2 and a control module 3.

The device 1 is obtained by mutually coupling the delivery module 2 and the control module 3. This coupling is reversible, i.e. the two modules 2 and 3 can be decoupled after having been coupled.

One or more gaskets (not illustrated) can be interposed between the delivery module 2 and the control module 3 in order to ensure the tightness of the mutual coupling.

In the embodiment described and illustrated herein, the control module 3 must be coupled to the delivery module 2 by a mechanical coupling in order to be able to deliver the medicament; in other possible embodiments, it is sufficient for the control module 3 to be temporarily positioned in proximity to the delivery module 2 (without any mechanical coupling between the two modules 2 and 3) for the medicament dose to be delivered.

The delivery module 2 is preferably disposable, while the control module 3 is preferably reusable. In other words, the delivery module 2 is configured to be applied on the body of the patient only once and for a certain period of time to deliver the medicament, completely or in part, in one or more subsequent injections, even temporally spaced apart. Conversely, the control module 3 can be used several times, by coupling it from time to time with a new delivery module 2.

In an embodiment of the device 1, the control module 3 is similar to the one described in patent application WO 2024116017 to the Applicant, which is incorporated herein by reference.

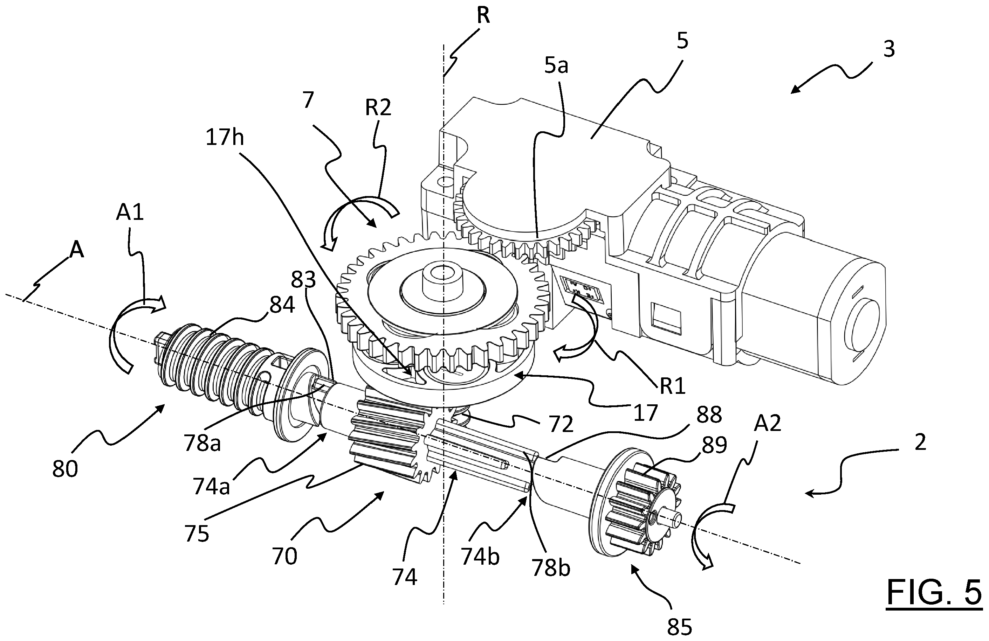

In particular, with reference to Figure 5, the control module 3 comprises a control unit (not illustrated) usually made in a printed circuit board and configured to control the delivery of the medicament from the delivery module 2, a primary or secondary power supply battery (not illustrated) configured to supply the control unit, and a motor 5 which controls in rotation a driving rotor 7 about an axis of rotation R.

The control module 3 also comprises a power switch (not illustrated) for activating the control unit and an activation button 9, which the patient presses to operate the device 1.

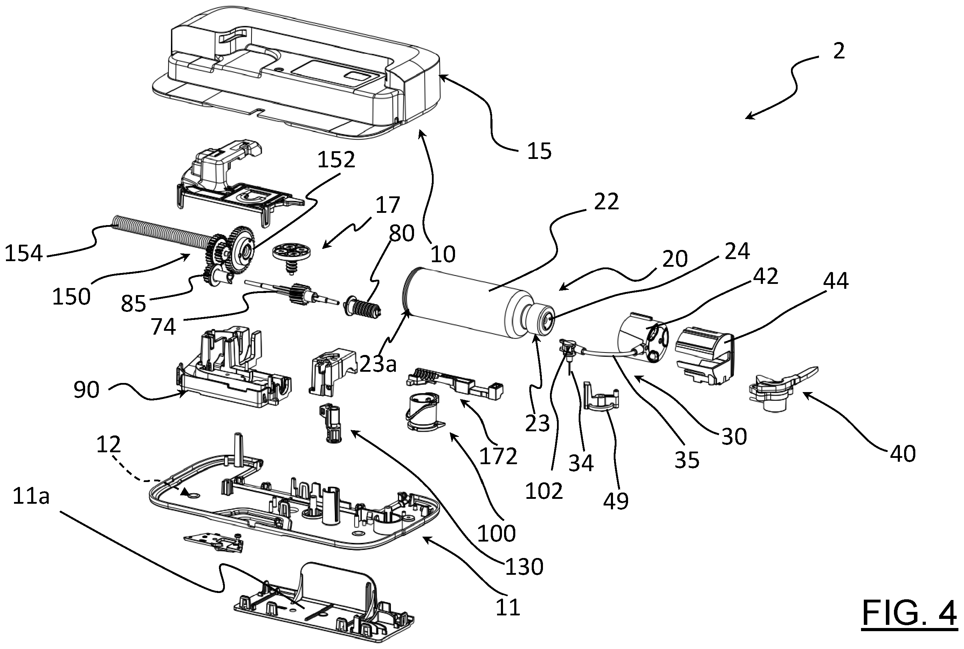

As illustrated in Figure 4, the delivery module 2 comprises a main body 11 and a cover 15 associated with the main body 11 and intended to be interposed between the main body 11 and the control module 3. Preferably, the cover 15 is welded to the main body 11 by laser welding.

A housing compartment 10 is defined between the main body 11 and the cover 15 for housing all the components of the delivery module 2.

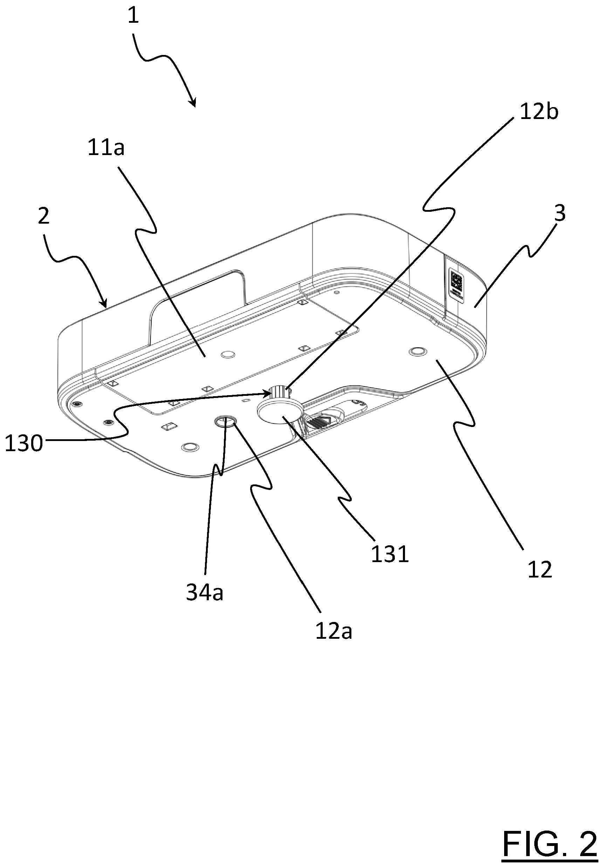

The main body 11 comprises an application surface 12 (also visible in Figure 2) configured to enter into contact with the patient's body. The application surface 12 is substantially flat and defines a resting plane P for the skin of the patient.

The application surface 12 comprises two through openings 12a, 12b (Figure 2), which will be discussed below.

Among the components housed in the housing compartment 10 there are a cartridge 20 and a fluidic path 30 (Figure 4).

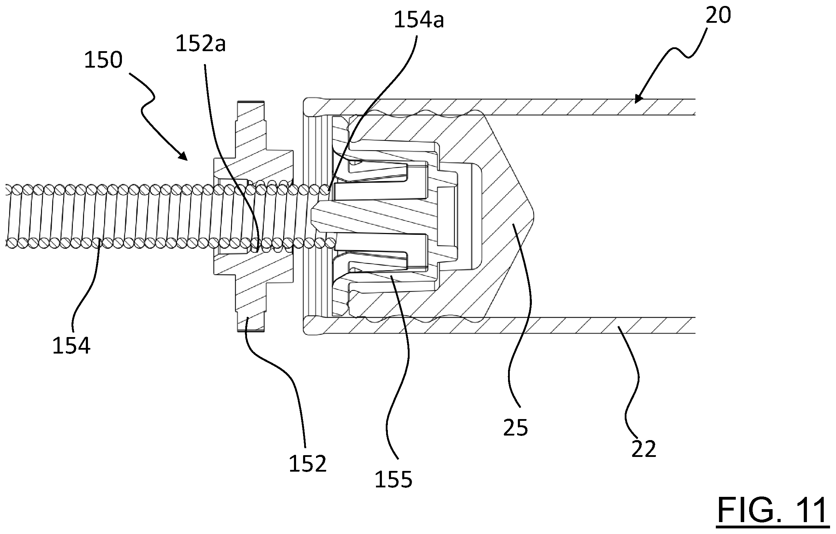

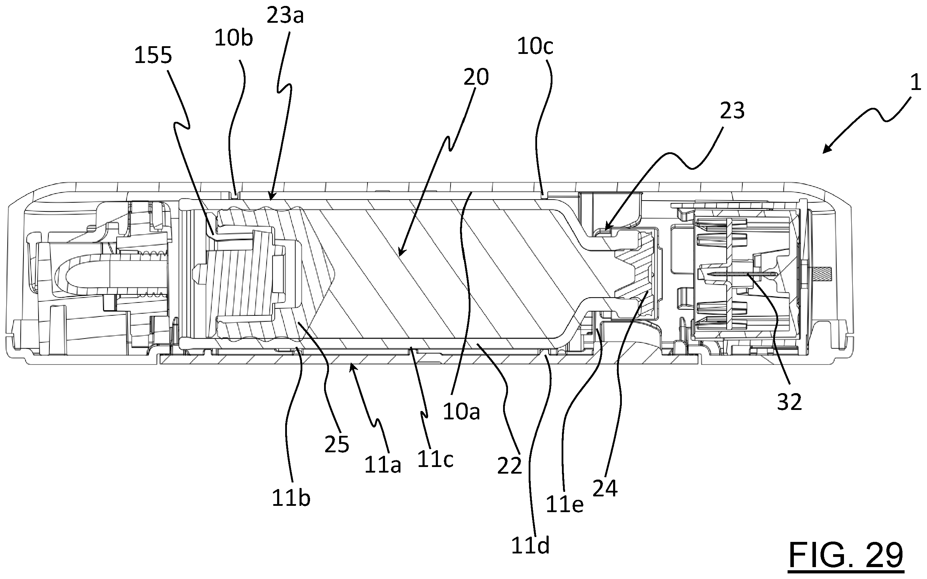

The cartridge 20 is housed in the housing compartment 10 and comprises a substantially cylindrical container 22 made of plastic or glass material and containing the medicament to be delivered. A pierceable septum 24, that closes the container 22 and guarantees the sterility thereof until the device 1 is used, is provided at one end portion 23 of the container 22. A plunger 25 (illustrated in Figures 11 and 29) is slidable in the container 22 to deliver the medicament.

Preferably, the cartridge 20 is pre-filled with the medicament to be delivered, is non-removable and non-refillable.

The fluidic path 30 is arranged on the main body 11 and is configured to be travelled by the medicament during the subcutaneous delivery of the medicament, allowing the movement of the medicament from the cartridge 20 to the body of the patient.

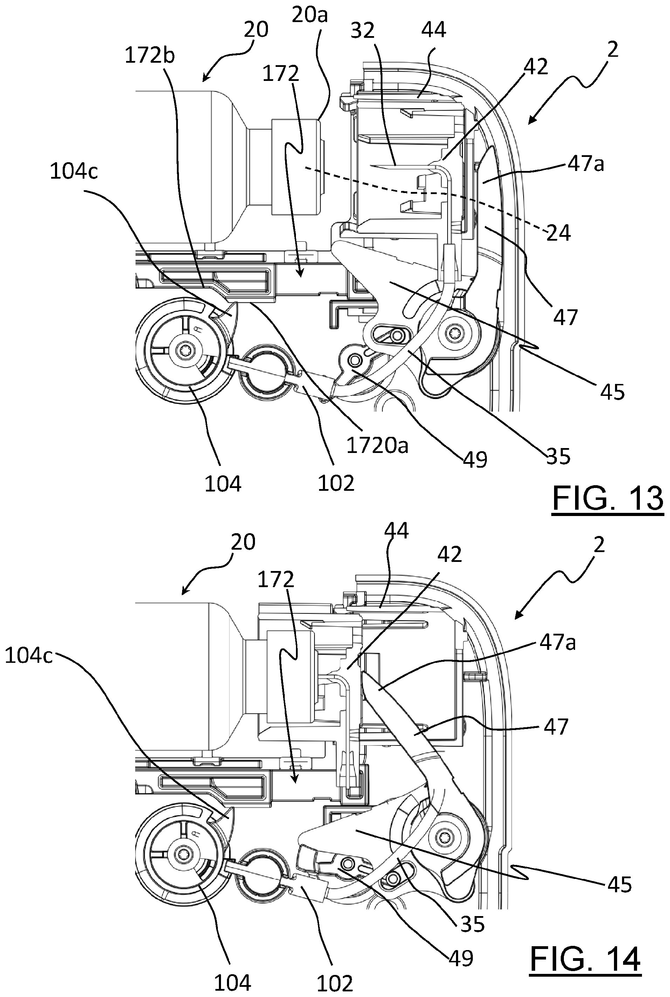

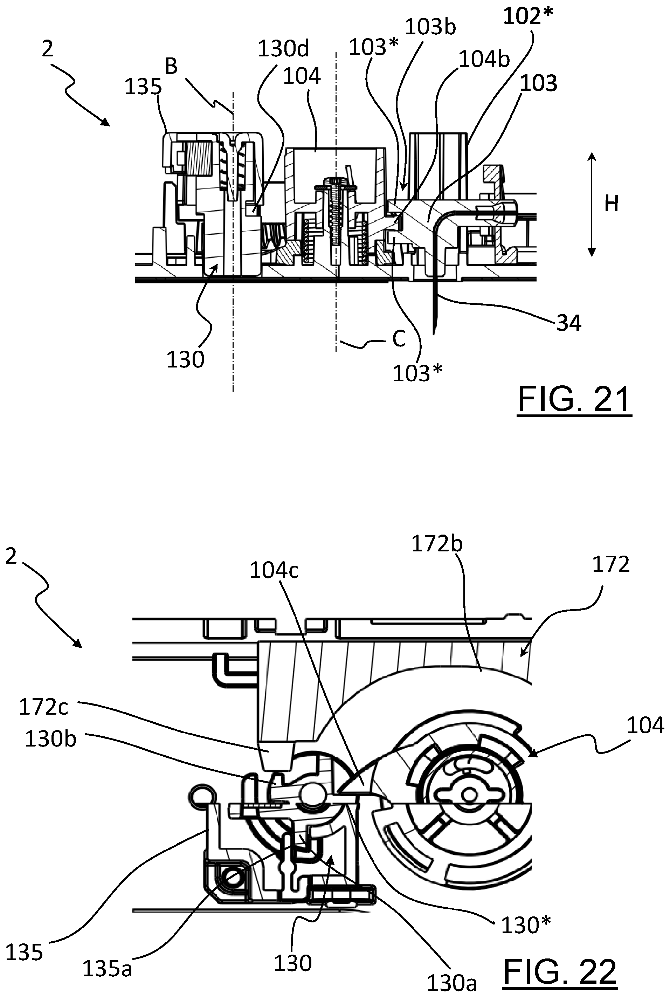

The fluidic path 30 comprises a piercing needle 32 (illustrated for example in Figure 13) configured to pierce the pierceable septum 24, an injection needle 34 (illustrated for example in Figure 21) configured to be inserted into the patient's body, and a flexible tube 35 (illustrated for example in Figure 4) that puts said needles 32 and 34 in fluid communication.

In particular, the flexible tube 35 has an end connected to a support member 42 that supports the piercing needle 32 and an opposed end connected to a support member 102 that supports the injection needle 34.

The delivery module 2 further comprises a fluidic path opening system 40 configured to command the piercing of the pierceable septum 24 by the piercing needle 32 and thereby put the container 22 in fluid communication with the injection needle 34 through the piercing needle 32 and the flexible tube 35.

The support member 42 is slidably mounted on a sliding guide 44 mounted on the main body 11. The support member 42 is movable between a first operating position in which the piercing needle 32 is spaced apart from the pierceable septum 24 (as illustrated for example in Figure 13) and the fluidic path 30 is closed, and a second operating position in which the piercing needle 32 has pierced the pierceable septum 24 (as illustrated for example in Figure 14) thereby opening the fluidic path 30.

As illustrated in Figures 4 and 5, the delivery module 2 comprises a driven rotor 17 mounted on the main body 11 and controlled in rotation by the driving rotor 7 provided in the control module 3.

In particular, the driven rotor 17 is substantially coaxial to the axis of rotation R and is controlled in rotation by the driving rotor 7 due to a magnetic coupling between the driven rotor 17 and the driving rotor 7.

When the delivery module 2 is coupled to the control module 3, the driving rotor 7 is thus arranged above the driven rotor 17 at a predetermined distance from the latter. This distance is for example 3 mm.

The motor 5 can control in rotation, via a gear 5a, the driving rotor 7 selectively in a first rotation direction R1 about the axis of rotation R (clockwise direction in Figure 5) and in a second rotation direction R2 (anticlockwise direction in Figure 5) opposite to the first rotation direction R1.

The driven rotor 17 is then controlled in rotation by the driving rotor 7 selectively in the first rotation direction R1 and in the second rotation direction R2 due to the interaction of the magnetic fields generated by the two rotors 7, 17.

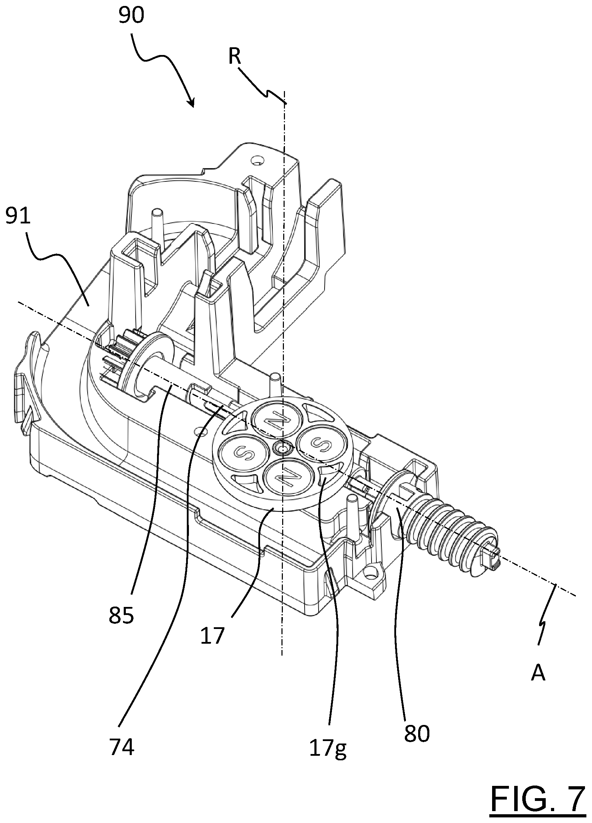

As illustrated in Figure 5, a motion transmission mechanism 70 is coupled to the driven rotor 17, also illustrated in Figure 7.

Such a motion transmission mechanism 70 is configured to transfer motion from the driven rotor 17 to a first motion transmission member 80, when the driven rotor 17 rotates in the first rotation direction R1, and from the driven rotor 17 to a second motion transmission member 85, when the driven rotor 17 rotates in the second rotation direction R2.

The first motion transmission member 80 and the second motion transmission member 85 are arranged on opposite sides with respect to the motion transmission mechanism 70.

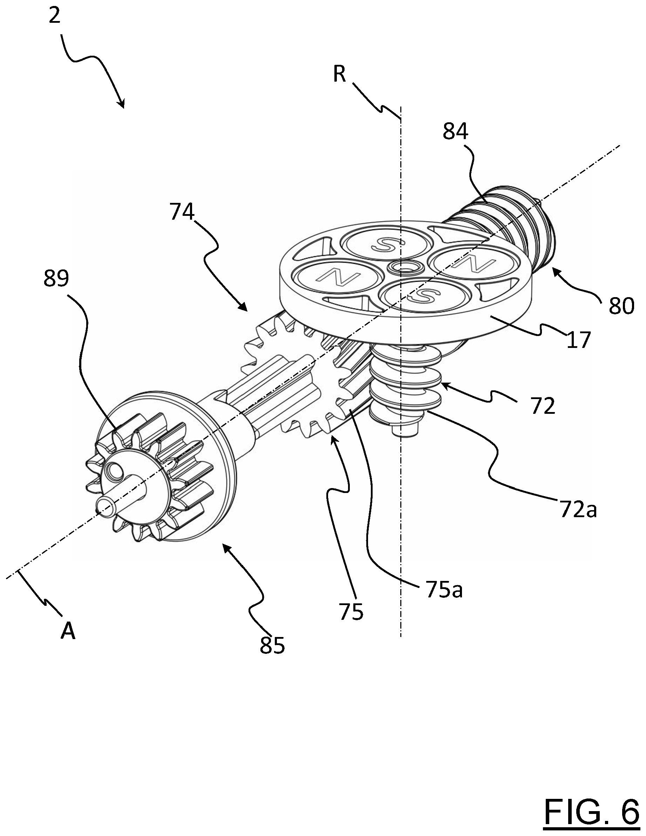

As illustrated in Figure 6, the motion transmission mechanism 70 comprises a worm screw 72 and a service shaft 74.

Figures 4, 5, 6, 7 and 10 illustrate a first embodiment of the service shaft 74.

The worm screw 72 is solidly associated with the driven rotor 17 and is substantially coaxial to the axis of rotation R. In the non-limiting example illustrated in the accompanying figures, the worm screw 72 is made as a single piece with the driven rotor 17.

The service shaft 74 extends along a longitudinal axis A and is axially movable selectively towards the first motion transmission member 80 and the second motion transmission member 85 (Figures 5, 6, 7 and 10). In the non-limiting example shown in the accompanying figures, the longitudinal axis A is substantially orthogonal to the axis of rotation R.

When the driven rotor 17 rotates in the first rotation direction R1, the service shaft 74 rotates about the longitudinal axis A in a first rotation direction A1 (e.g., clockwise direction in Figure 5) and translates along the longitudinal axis A towards the first motion transmission member 80 until it is coupled with the latter and decoupled from the second motion transmission member 85. When, on the other hand, the driven rotor 17 rotates in the second rotation direction R2, the service shaft 74 rotates about the longitudinal axis A in a second rotation direction A2 opposite the first rotation direction A1 (e.g., anticlockwise in Figure 5) and translates along the longitudinal axis A towards the second motion transmission member 85 until it is coupled with the latter and decoupled from the first motion transmission member 80.

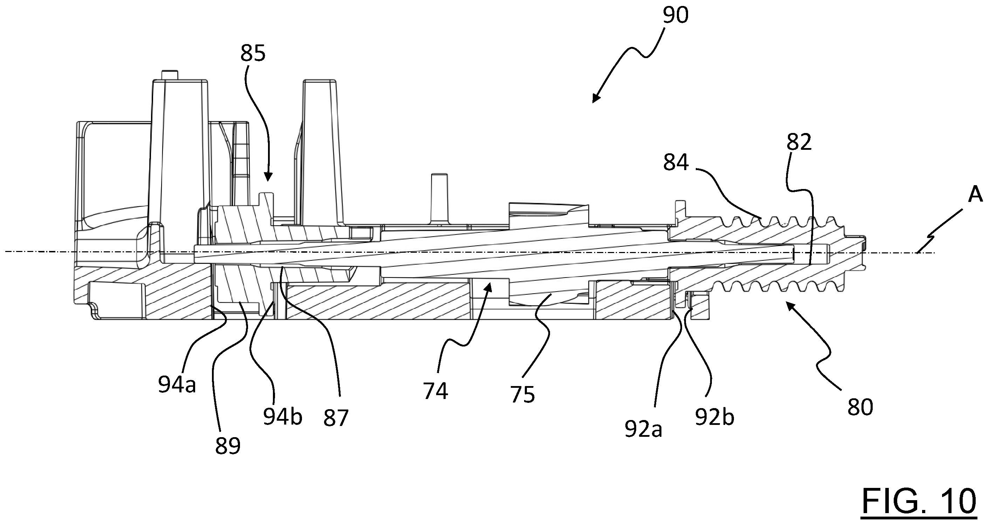

The transmission of motion from the driven rotor 17 to the service shaft 74 occurs by the engagement of the worm screw 72 with a toothed wheel 75 solidly associated with the service shaft 74 and substantially coaxial to the longitudinal axis A (Figure 6). In the non-limiting example illustrated in the accompanying figures, the toothed wheel 75 is made as a single piece with the service shaft 74 (Figure 10).

In particular, with reference to Figure 6, the worm screw 72 comprises a first helical toothing 72a and the toothed wheel 75 comprises a second helical toothing 75a conjugated to the first helical toothing 72a and engaged with the latter.

In the non-limiting example shown in Figures 5 and 6, the worm screw 72 has a thread and the first helical toothing 72a is sized in such a way that at each revolution of the worm screw 72 the next tooth of the second helical toothing 75a is engaged.

Preferably, the toothed wheel 75 has a module comprised between 0.3 mm and 1 mm, for example equal to 0.5 mm, and a number of teeth comprised between 12 and 25, for example equal to 15.

The first helical toothing 72a preferably has a primitive diameter comprised between 0.5 mm and 11.4 mm, for example equal to 3.6 mm, while the second helical toothing 75a preferably has a primitive diameter comprised between 3.6 mm and 28.9 mm, for example equal to 7.6 mm.

The second helical toothing 75a preferably has a number of teeth comprised between 12 and 25. In the non-limiting example shown in the accompanying figures, the second helical toothing 75a has 15 teeth.

Preferably, the second helical toothing 75a is inclined with respect to a plane containing the axis of rotation of the toothed wheel 75, that is the aforementioned longitudinal axis A, by an angle comprised between 5° and 30°. In the non-limiting example shown in the accompanying figures, this angle is equal to 7.89°.

Again with reference to Figures 5, 6 and 10, the service shaft 74 comprises a first end portion 74a and a second end portion 74b opposite the first end portion 74a. The first end portion 74a is configured to be rotationally coupled to the first motion transmission member 80 when the driven rotor 17 rotates in the first rotation direction R1 and the second end portion 74b is configured to be rotationally coupled to the second motion transmission member 85 when the driven rotor 17 rotates in the second rotation direction R2.

In particular, when the first end portion 74a is rotationally coupled to the first motion transmission member 80, the first end portion 74a is housed in a first axial hole 82 formed in the first motion transmission member 80 and a first abutment element 83 formed in the first motion transmission member 80 cooperates in abutment with a first thrust element 78a formed in the first end portion 74a, causing the first motion transmission member 80 to rotate in the first rotation direction A1.

When, on the other hand, the second end portion 74b is rotationally coupled to the second motion transmission member 85, the second end portion 74b is housed in a second axial hole 87 formed in the second motion transmission member 85 and a second abutment element 88 formed in the second motion transmission member 85 cooperates in abutment with a second thrust element 78b formed in the second end portion 74b, causing the second motion transmission member 85 to rotate in the second rotation direction A2.

In the embodiment illustrated in the accompanying figures, the first axial hole 82 is a blind hole and the second axial hole 87 is a through hole.

In alternative embodiments of the invention (not illustrated), the rotational coupling between said first end portion 74a and the first motion transmission member 80 and/or between said second end portion 74b and the second motion transmission member 85 may be achieved by other types of couplings, such as by mechanical or viscous friction coupling, key coupling, coupling with deformation of elastic means, rack and pinion coupling, etc.

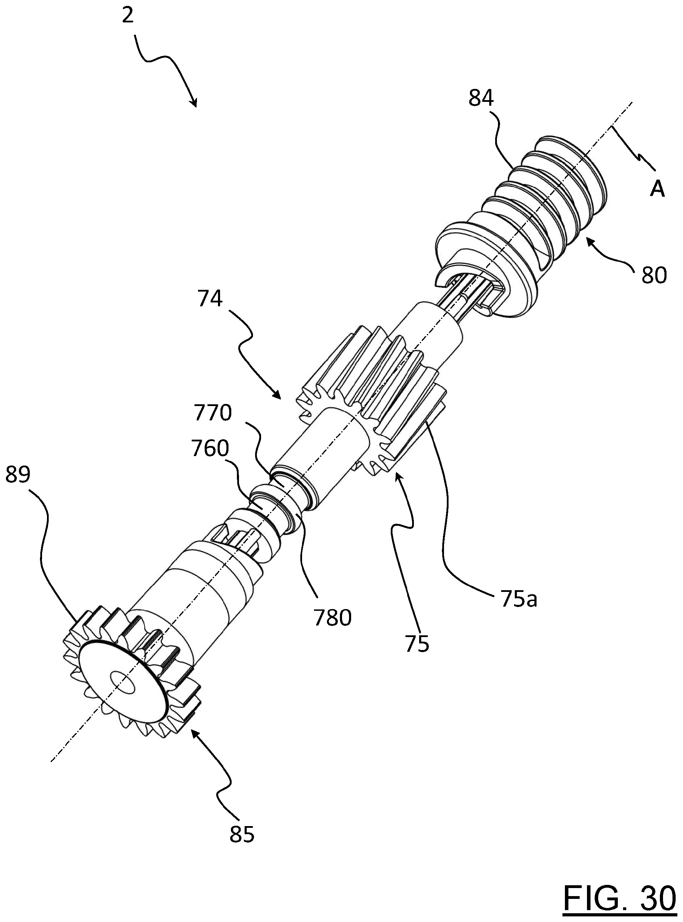

Figure 30 illustrates a second embodiment of the service shaft 74, which differs from the one illustrated in Figures 4, 5, 6, 7 and 10 mainly in that it comprises, between the two end portions 74a and 74b, a pair of annular grooves 760, 770 separated by an annular flange 780.

Each of the two annular grooves 760, 770 comprises a connecting surface 761, 771 distal from the annular flange 780. This connecting surface is preferably flat or curved.

The annular flange 780 preferably has a radially outer surface 781 that is curved and opposing side surfaces 782a, 782b that are substantially flat.

As illustrated in Figure 7, the motion transmission mechanism 70 is housed within a pre-assembled unit 90.

The pre-assembled unit 90 comprises a support body 91.

The first motion transmission member 80 and the second motion transmission member 85 are supported by the support body 91 of the pre-assembled unit 90 and in axial abutment against the respective inner walls 92a, 92b and 94a, 94b of the support body 91.

The worm screw 72 is in abutment against a further inner wall (not illustrated) of the support body 91.

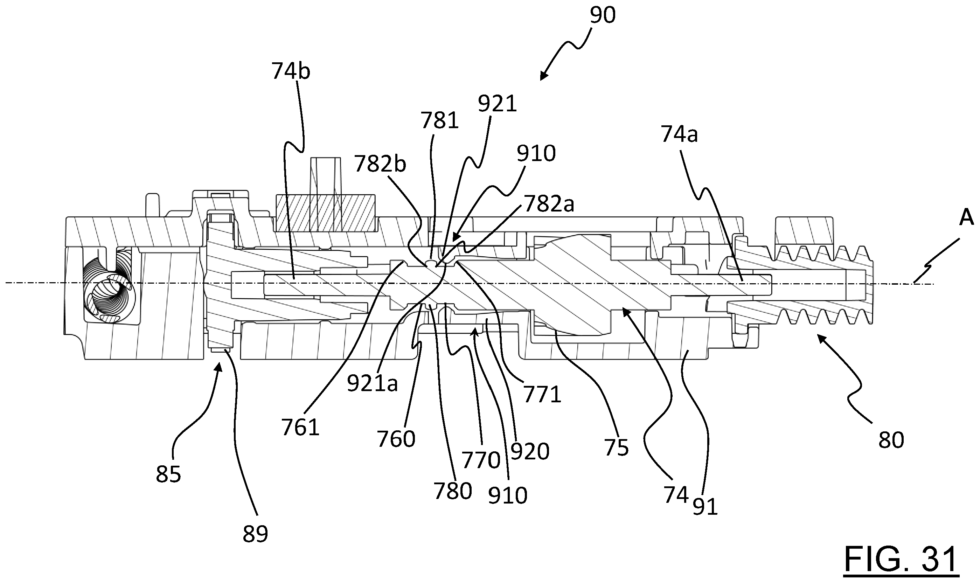

Figure 31 shows an embodiment of the support body 91 that is used in combination with the service shaft 74 of Figure 30.

In this embodiment, the support body 91 comprises two elastically deformable coupling elements 910 arranged on opposing sides with respect to the service shaft 74 and in proximity to the pair of annular grooves 760, 770.

Each coupling element 910 comprises an arm 920 connected to the support body 91 and having a free end portion 921 which, in the rest condition illustrated in Figure 31 (corresponding to that in which the service shaft 74 is coupled to the second motion transmission member 85) is housed in the annular groove 770. When, on the other hand, the service shaft 74 is coupled to the first motion transmission member 80, the free end portion 921 of each arm 920 is housed in the annular groove 760.

The transition of the free end portion 921 of each arm 920 from the annular groove 770 to the annular groove 760 takes place when the service shaft 74 changes rotation direction for passing from the condition in which it is coupled to the second motion transmission member 85 to that in which it is coupled to the first motion transmission member 80. In this case, the service shaft 74 moves axially with respect to the support body 91 away from the second motion transmission member 85 and towards the first motion transmission member 80.

During this axial movement, the annular flange 780 comes into contact with the free end portion 921 of each arm 920 and causes the latter to bend elastically. Specifically, the annular flange 780 pushes the free end portions 921 of the two arms 920 away from each other until the annular flange 780 passes those free end portions 921. At this point, the two arms 920 return to their rest position, resulting in the free end portions 921 being housed in the annular groove 760.

It goes without saying that a subsequent change of the rotation direction of the service shaft 74 from the condition in which it is coupled to the first motion transmission member 80 to that in which it is coupled to the second motion transmission member 85 results in an elastic bending of the arms 920 similar to that described above and in the consequent passage of the free end portion 921 of each arm 920 from the annular groove 760 to the annular groove 770.

Due to the fact that the radially outer surface 781 of the annular flange 780 is, preferably, curved, the annular flange 780 exerts a continuous and incremental pressure on the arms 920 during the transition of the free end portion 921 of each arm 920 from the annular groove 770 to the annular groove 760 and vice versa. Consequently, the above-mentioned transition takes place gradually.

The free end portion 921 of each arm 920 has a radially inner surface 921a that is curved or slightly pointed.

In particular, the profile of the radially inner surface 921a and the connecting surfaces 761 and 771 of the annular grooves 760 and 770 is such as to avoid any mutual contact of said surfaces during the transition of the free end portions 921 of the arms 920 from the annular groove 760 to the annular groove 770 (and vice versa) and after such transition. Indeed, such a contact would result in an undesirable friction during the rotation of the service shaft 74.

For the same reason, the axial width and depth of the annular grooves 760 and 770, as well as the maximum axial dimension and the maximum radial dimension of the free end portion 921 of each arm 920, is appropriately selected to avoid an undesirable mutual contact between the annular grooves 760 and 770 and the free end portion 921 of the arms 920 when the latter are housed in the annular groove 760 or in the annular groove 770.

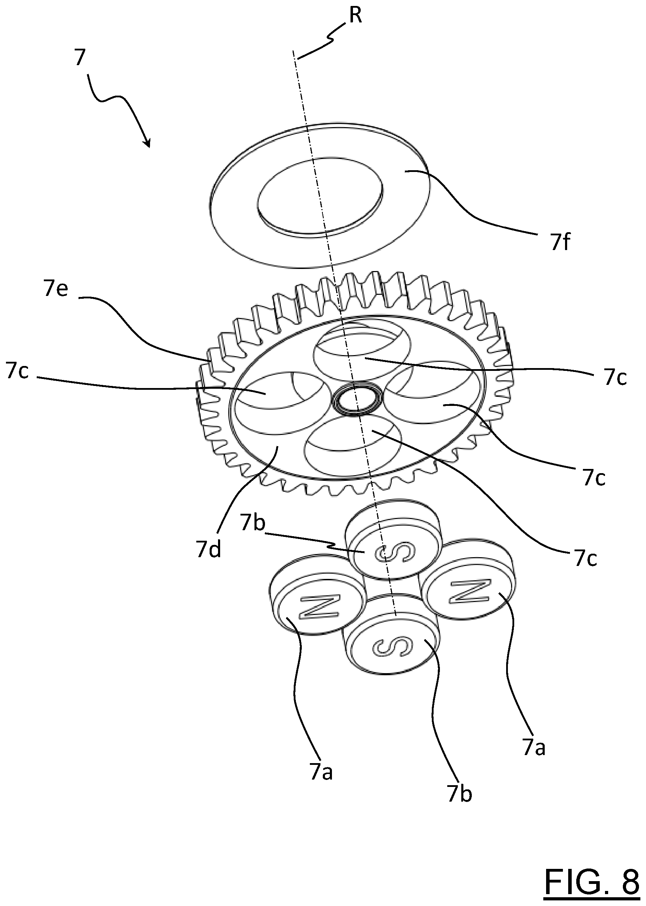

With reference to Figure 8, the driving rotor 7 comprises a pair of permanent magnets 7a having a first polarity (e.g. north polarity, indicated by the letter "N") and a pair of permanent magnets 7b having a second polarity (e.g. south polarity, indicated by the letter "S") opposite the first polarity. For example, the permanent magnets 7a, 7b are of the NdFeB type, namely made of an alloy of neodymium, iron and boron.

The magnets 7a and 7b are arranged circumferentially one after the other around the axis of rotation R. They are housed in respective seats 7c formed in a body 7d of the driving rotor 7. In the non-limiting example illustrated in the accompanying figures, the magnets 7a, 7b are substantially cylindrical in shape and the seats 7c are defined by respective through holes formed in the body 7d and having a substantially cylindrical shape conjugated to that of the magnets 7a, 7b.

Each magnet 7a is circumferentially interposed between two respective magnets 7b, resulting in an alternating circumferential arrangement of magnets 7a and magnets 7b.

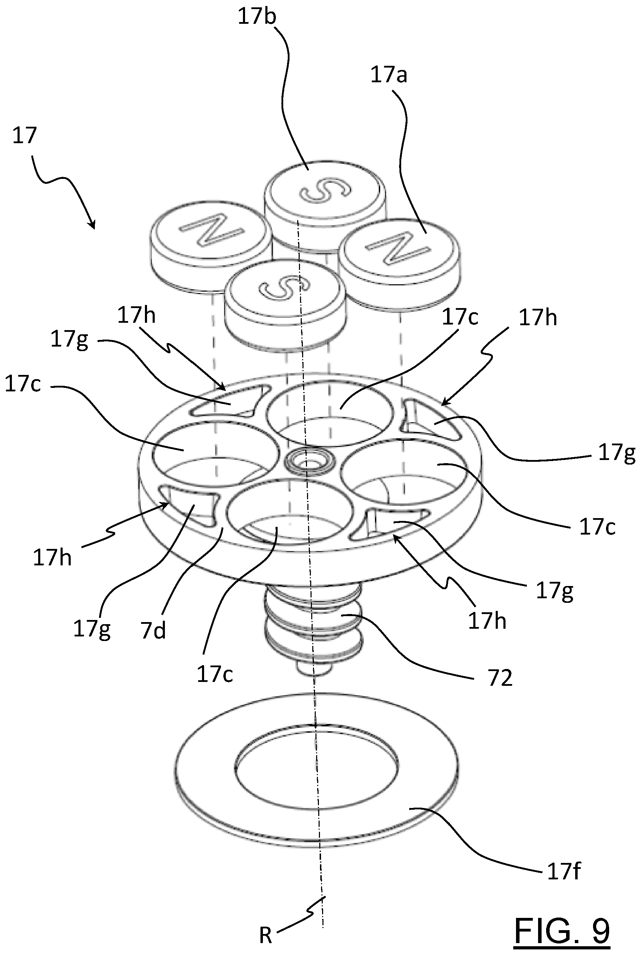

As illustrated in Figures 8 and 9, the driving rotor 7 and the driven rotor 17 each comprise a respective ferromagnetic ring 7f, 17f arranged on the opposite side with respect to the other rotor 17, 7 when the driving rotor 7 is arranged above the driven rotor 17.

With reference to Figure 9, the driven rotor 17 comprises a pair of permanent magnets 17a having a first polarity (e.g. north polarity, indicated by the letter "N") and a pair of permanent magnets 17b having a second polarity (e.g. south polarity, indicated by the letter "S") opposite the first polarity. For example, the magnets 17a, 17b are also of the NdFeB type.

The magnets 17a and 17b are arranged circumferentially one after the other around the axis of rotation R, housed in respective seats 17c formed in a body 17d of the driven rotor 17. In the non-limiting example shown in the accompanying figures, the permanent magnets 17a, 17b are substantially cylindrical in shape and the seats 17c are through openings formed in the body 17d and having a substantially cylindrical shape conjugated to that of the magnets 17a, 17b.

In the non-limiting example shown in the accompanying figures, the magnets 17a, 17b are identical to the magnets 7a, 7b.

Each magnet 17a is circumferentially interposed between two respective magnets 17b, resulting in an alternating circumferential arrangement of magnets 17a and magnets 17b.

In the non-limiting example illustrated in the accompanying figures, the magnets 7a, 7b and the ferromagnetic ring 7f of the driving rotor 17 and the magnets 17a, 17b and the ferromagnetic ring 17f of the driving rotor 17 are not attached to the body 7d and the body 17d respectively by mechanical attachments or glue. In fact, they are sized in such a way that the magnetic force exerted between them is sufficient to keep them in a predetermined position with respect to the respective bodies 7d, 17d. This simplifies the assembly operations of the device 1.

For example, the bodies 17d, 7d are made of injection-moulded plastic. They are essentially cylindrical in shape and similar in size.

The body 7d comprises a circumferential toothing 7e engaged to the gear 5a of the motor 5.

The body 17d comprises a plurality of lightening through openings 17g. In the non-limiting example illustrated in Figure 9, there are four lightening through openings 17g, each arranged between a respective pair of seats 17c. Similar lightening through openings can also be provided in the body 7d.

Figures 5-9 illustrate an arrangement between the driving rotor 7 and the driven rotor 17 that allows a stable equilibrium condition to be achieved and maintained between the rotors 7, 17. In this arrangement, each magnet 7a and 7b of the driving rotor 7 is arranged above a respective area 17h defined between a respective magnet 17a and a respective magnet 17b of the driven rotor 17. In practice, each of the magnets 7a, 7b is arranged above a respective lightening through opening 17g.

In the above arrangement, the rotors 7, 17 are angularly offset from each other by 45° using a magnet 7a and a magnet 17a as reference. In this way, the axial force generated between the rotors 7, 17 is minimised, while the shear forces generated by the magnetic field present at the rotors 7, 17 are maximised, thus maximising the torque that can be transmitted between the rotors 7, 17.

The ferromagnetic rings 7f, 17f increase this effect of maximising the torque transmitted between the rotors 7, 17. Indeed, they short-circuit the magnetic flux generated, preventing it from being dispersed in the air and concentrating it in the area between the two rotors 7, 17.

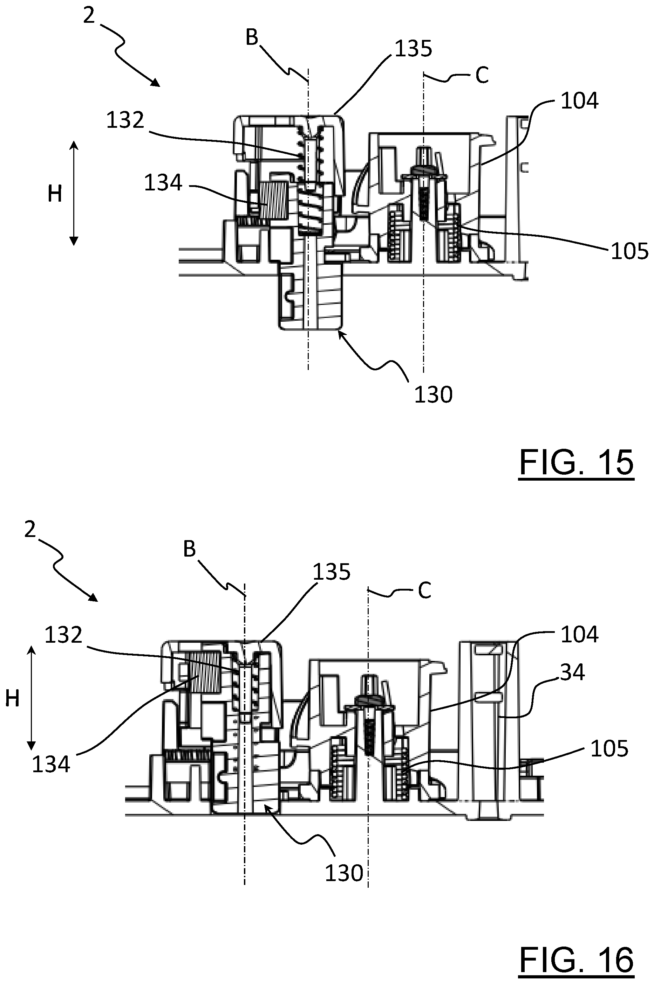

With reference to Figures 15 and 16, the device 1 comprises a position sensor 130 adapted to detect contact of the device 1 with the patient's skin.

The position sensor 130 is movable, through the opening 12b formed on the application surface 12, between a first operating position (Figure 15) in which it protrudes from the device 1 and a second operating position (Figure 16) in which it does not protrude from the device 1.

The position sensor 130 is subjected to a thrust action exerted by an elastic element 132, in particular by a compression spring. This thrust action tends to keep the position sensor 130 in the first operating position both before the device 1 is applied to the patient's skin and when the device 1 is removed from the patient's skin, and to allow the position sensor 130 to reach the second operating position when the device 1 is applied to the patient's skin.

The position sensor 130 comprises a magnet 134 and a Hall-effect sensor (not illustrated) arranged in the control module 3 in proximity to the magnet 134. In Figure 2 an optional plate 131 is illustrated, which is attached to an end portion of the position sensor 130 facing the patient's skin.

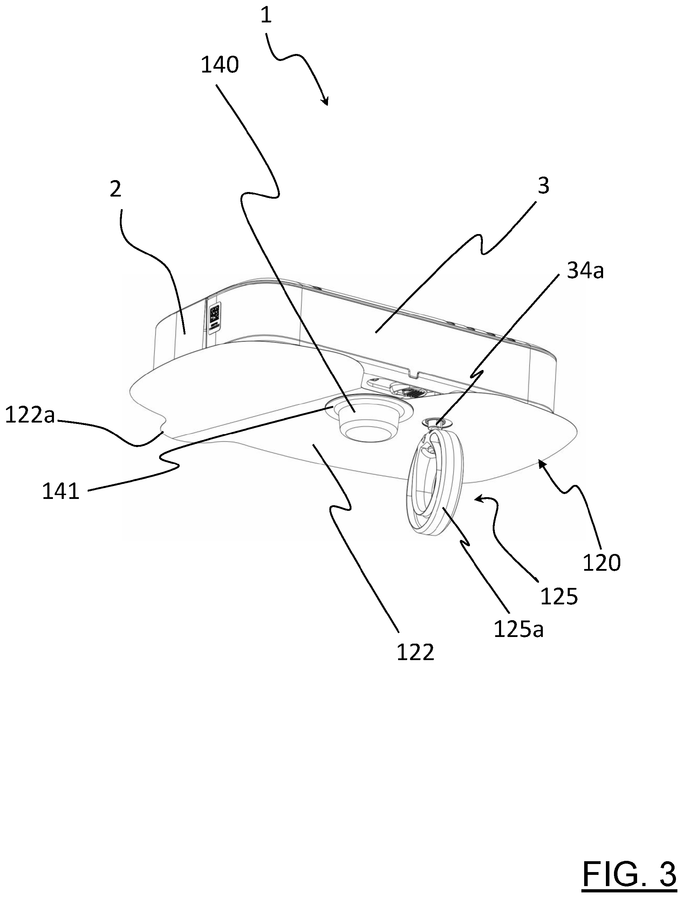

The patient is provided with a ready-to-use device 1, which includes a patch 120 applied to the application surface 12 of the main body 11 of the delivery module 2, as shown in Figure 3.

The patch 120 comprises an adhesive layer (not illustrated) solidly attached to the application surface 12 and a protective layer 122 removably attached to the adhesive layer. Before carrying out the administration of the therapy, the protective layer 122 is removed and the device 1 is attached to the skin of the patient through the adhesive layer.

The adhesive layer is adequately sized to support the weight of the device 1 during therapy and to follow the potential movements of the patient during therapy.

The adhesive layer could be an acrylic, silicone or hydrogel adhesive layer. The Applicant preferably selected an acrylic adhesive layer. The adhesive layer is supported by a non-woven fabric substrate.

The patch 120 can be attached to the device by gluing, welding or by interposition of an adhesive layer, such as an acrylic adhesive layer.

As shown in Figure 3, the device 1 further comprises a removal member 125 for removing a cap 34a which protects the injection needle 34 and an additional cap 140 which protects the position sensor 130.

The removal member 125 comprises a gripping portion 125a configured to be gripped by the user's (or patient's) fingers and a coupling portion (not visible in the accompanying figures) coupled to the cap 34a.

The cap 140 is solidly attached to the protective layer 122 of the patch 120 and comprises a collar 141 attached to the protective layer 122 by the interposition of a double-sided adhesive element.

The cap 140 is made of a rigid material and is shaped to house with play and completely the position sensor 130 when the latter is in its first operating position.

In the embodiment illustrated herein, the cap 140 has a frustoconical shape.

Initially the position sensor 130 is in the first operating position and the cap 140 covers the position sensor 130, as shown in Figure 3.

Prior to applying the device 1 to the patient's skin, the user or patient removes the cap 34a from the injection needle 34 by acting on the gripping portion 125a of the removal member 125. Next, the protective layer 122 is removed by acting on a flap 122a of the latter, simultaneously removing from the protective layer 122 also the cap 140 and thereby releasing the position sensor 130 and the adhesive layer of the patch 120.

Then the device 1 can be applied on the patient's skin by attaching it through the adhesive layer of the patch 120. The patient's skin counteracts the pushing action exerted by the elastic element 132 and makes the position sensor 130 retract inside the delivery module 2, bringing the magnet 134 closer to the Hall effect sensor and thus allowing the control unit to establish that the device 1 is in contact with the patient's skin and to activate the administration of the therapy.

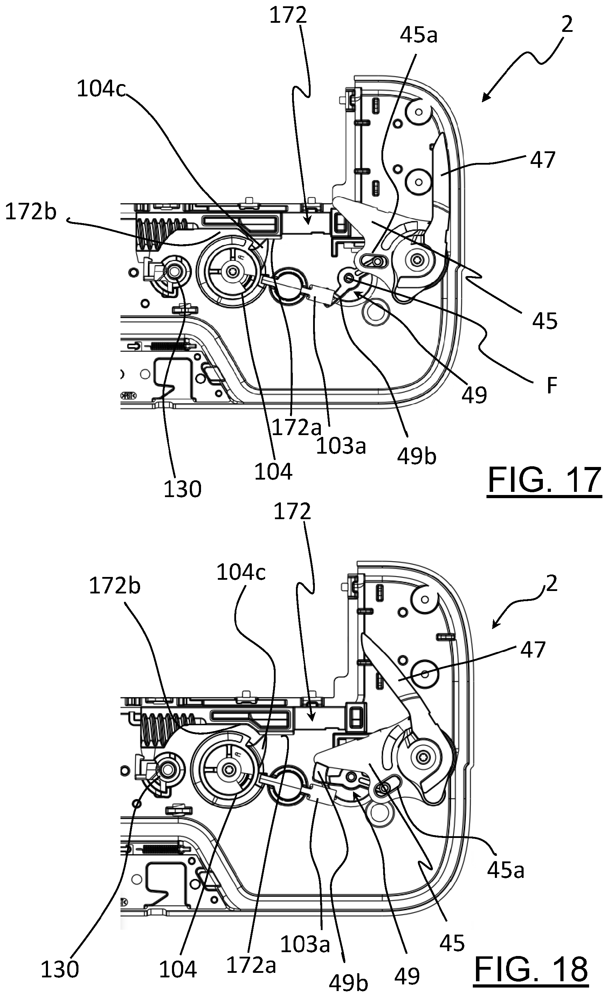

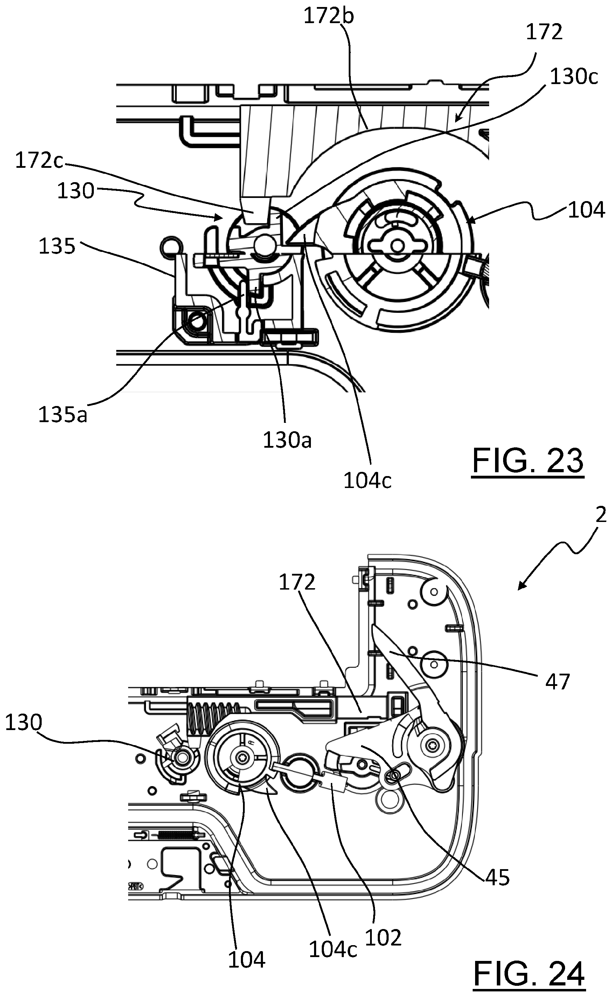

As illustrated in Figure 4 and Figures 17-21, the delivery module 2 also comprises a mechanism 100 for moving the support member 102 of the injection needle 34. Such a mechanism 100 is activated, upon starting the therapy administration, to move the injection needle 34 through the through opening 12a from a rest position, in which the injection needle 34 is entirely arranged inside the device 1 and does not protrude from the application surface 12 of the device 1 (Figures 17 and 18), to an injection position in which the injection needle 34 protrudes at least partially from the application surface 12 of the device 1 (Figure 21). The aforementioned mechanism 100 is also activated, at the end of therapy administration, to move the injection needle 34, again through the through opening 12a, from the injection position to the rest position (Figure 24).

The administration of therapy comprises the controlled movement of the plunger 25 within the container 22 towards the pierceable septum 24. For this purpose, the delivery unit 2 comprises a medicament delivery mechanism 150 (Figures 4 and 11) which is activated, upon command of the control unit, by the motion transmission mechanism 70 by rotation of the second motion transmission member 85.

In the embodiment illustrated in the accompanying drawings, the second motion transmission member 85 comprises a toothed wheel 89 engaged in turn to a toothed wheel 152. As illustrated in Figure 11, the latter comprises a toothed through axial cavity 152a engaged to the coils of a pre-stressed flexible spring 154 associated with the plunger 25. The rotation of the toothed wheel 152 causes the linear displacement of the flexible spring 154 and, consequently, the translation of the plunger 25 inside the container 22.

The flexible spring 154 is housed inside the main body 11 of the delivery module 2, specially curved so as to minimise the impact on the overall length of the device 1.

A spacer 155 is interposed between the plunger 25 and an end 154a of the flexible spring 154 proximal to the plunger 25. The spacer 155 is pre-assembled inside the cartridge 20 and rests on a large surface of the plunger 25, so that the load generated by the end 154a is evenly distributed over the plunger 25.

In the embodiment illustrated in the accompanying drawings, the first motion transmission member 80 comprises a worm screw 84.

This worm screw 84 engages a rack 172 (Figure 4). The latter is configured to activate the fluidic pathway opening system 40 and the mechanism 100 for moving the injection needle 34 in order to extract the latter from the device 1 prior to the therapy administration and to retract the latter into the device 1 after the therapy administration.

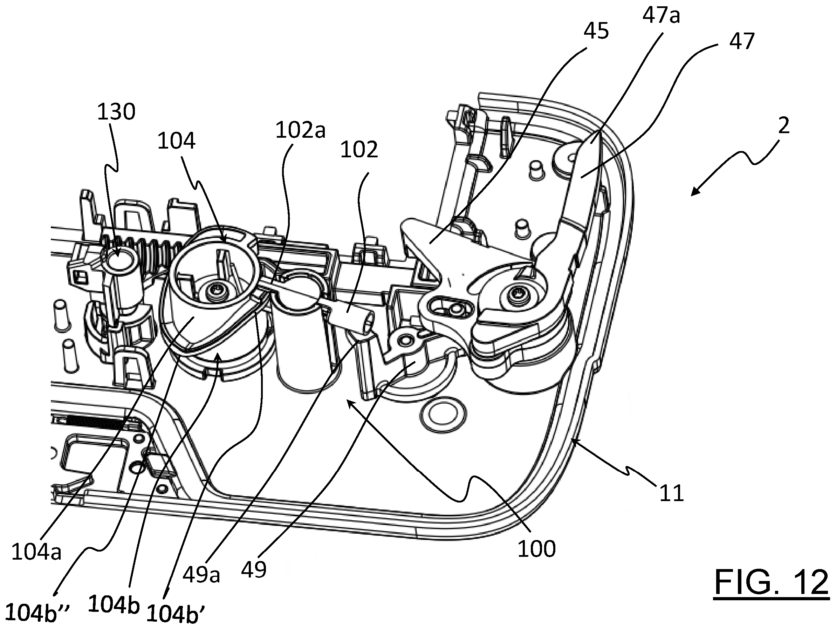

Activation of the fluidic pathway opening system 40 takes place as described below with reference to Figures 12-14.

The rotation of the worm screw 84 causes a linear displacement (or translation) of the rack 172 along a direction parallel to the longitudinal direction of the cartridge 20. This linear displacement unlocks the rotation of a first lever 45 under the thrust action of a torsion spring (not shown). The first lever 45 is integrally associated with a second lever 47, which thus also begins to rotate.

During such rotation, an end portion 47a of the second lever 47 contacts the support member 42 of the piercing needle 32 and pushes it towards the cartridge 20, thereby moving the piercing needle 32 towards the pierceable septum 24.

The movement of the support member 42 stops, after the piercing needle 32 has pierced the pierceable septum 24, due to an abutment between the support member 42 and an abutment surface 20a of the cartridge 20 (Figures 13 and 14).

Activation of the mechanism 100 for moving the injection needle 34 in order to extract it from the device 1 is performed as described below with reference to Figures 15-20.

In the initial configuration of the device 1, the position sensor 130 protrudes from the device 1 (Figure 15). After the device 1 is positioned on the patient's skin, the position sensor 130 is retracted into the device 1 and its rotation about its longitudinal axis B is blocked by a cover 135 placed on top of the position sensor 130 (Figure 16), as will be described below with reference to Figure 22.

The injection needle 34 is retracted into the device 1.

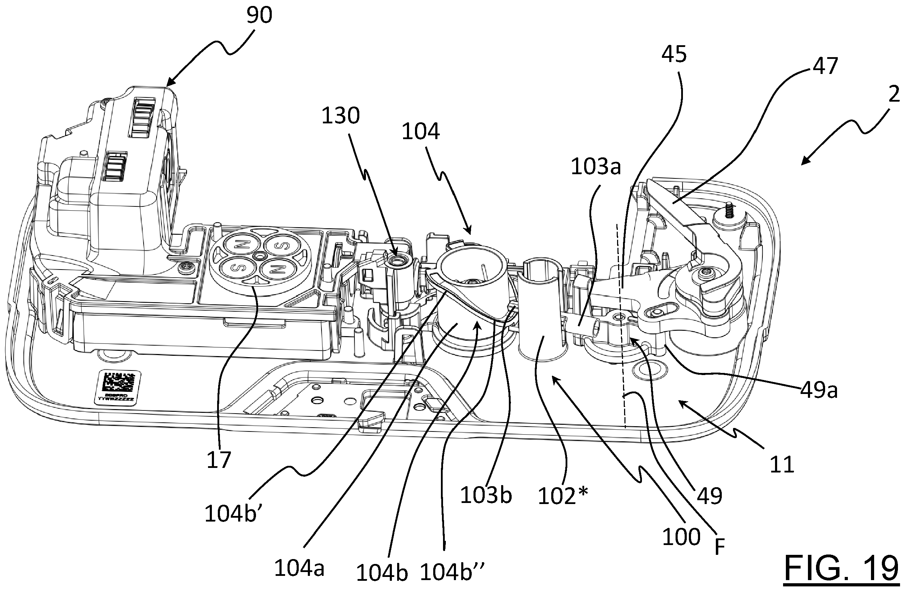

The first lever 45 has a slot 45a (Figures 17 and 18) within which the top of an end portion 49a (Figure 19) of a third lever 49 is arranged. The latter is pivoted on a base surface of the main body 11 at a pivoting axis F (Figures 17 and 19) and comprises, on the opposite side to the end portion 49a, an end portion 49b (Figure 17).

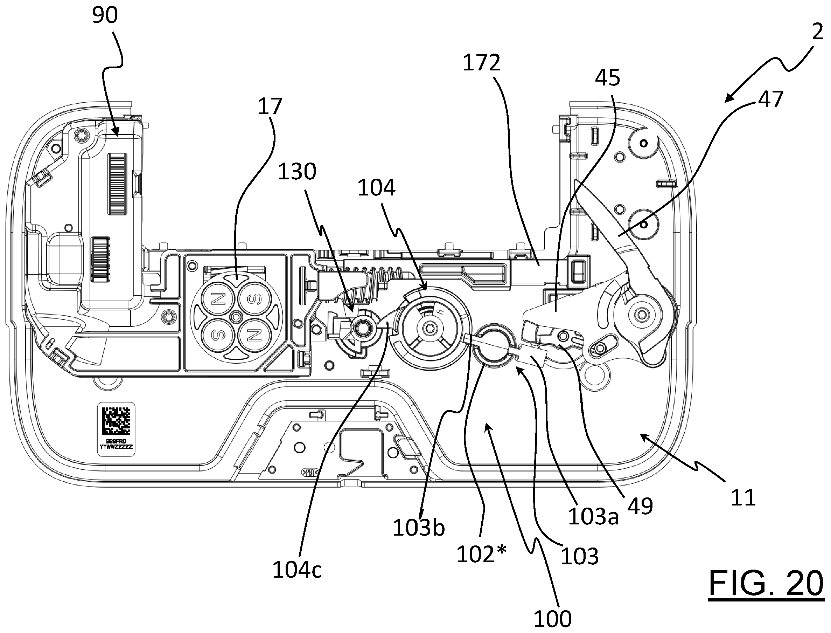

The support member 102 comprises a body 103 (Figure 21) movable along a direction H inside a guide cylinder 102* (Figures 19 and 21) attached to the base surface of the main body 11. The body 103 has, at a top portion thereof, opposing free end portions 103a, 103b (Figures 19 and 20).

The displacement of the support member 102, and thus of the injection needle 34, is prevented by the fact that the end portion 103a is arranged above the end portion 49b of the third lever 49 (Figure 17).

A control member 104 is configured to move the support member 102 along the direction H between the rest position (injection needle 34 arranged inside the delivery module 2) and the injection position (injection needle 32 protruding from the delivery module 2).

The control member 104 has a substantially cylindrical side wall 104a and is controlled in rotation about an axis of rotation C orthogonal to the application surface 12 by a preloaded torsion spring 105 (Figures 15 and 16).

The control element 104 comprises a cam profile 104b which protrudes externally from the substantially cylindrical side wall 104a (Figure 12). The cam profile 104b has opposing end portions 104b' arranged at or in proximity to a top surface of the control member 104 and a central portion 104b" arranged at or in proximity to a bottom portion of the control member 104.

The free end portion 103b of the body 103 of the support member 102 comprises a pair of appendages 103* which cooperate with the cam profile 104b on opposing sides thereof (Figure 21).

In the initial configuration of the device 1, the appendages 103* are arranged at one of the end portions 104b' of the cam profile 104b.

The rotation of the control member 104 is blocked by the rack 172 due to the abutment of a tooth 104c protruding externally from the substantially cylindrical side wall 104a against a flat surface portion 172a of the rack 172 (Figure 17).

As illustrated in Figure 18, the linear movement of the rack 172 causes the rotation of the first lever 45 and the third lever 49. The rotation of the latter results in the displacement of the top of the end portion 49a of the lever 49 inside the slot 45a and, consequently, the disengagement of the end portion 49b from the free end portion 103a of the body 103 of the support member 102.

The rack 172 is further displaced as a result of the further rotation of the worm screw 84 of the first motion transmission member 80 and the tooth 104c is located at a recessed portion 172b of the rack 172, no longer being in contact with the rack 172 (configuration immediately following the one illustrated in Figure 18). The torsion spring 105 thus brings in rotation the control member 104 until the tooth 104c comes into abutment with a first projecting portion 130* of the position sensor 130 (Figure 22), which, following the positioning of the device 1 on the patient's skin, has retracted into the device 1 (Figures 19 and 20). During this rotation, the pair of appendages 103* of the body 103 of the support member 102 moves following the cam profile 104b until it reaches the central portion 104b", causing the support member 102 to translate along the direction H until the injection needle 34 is extracted from the device 1 and penetrated into the patient's skin (Figure 21).

It is now possible to proceed with the administration of therapy.

There may or may not be a time interval between activation of the fluidic pathway opening system 40 and extraction of the injection needle 34. The time interval is provided in those cases in which the activation sequence of the device 1 also comprises a step in which air contained in the fluidic pathway 30 is released (priming step).

At the end of the therapy administration, the mechanism 100 for moving the injection needle 34 is activated to retract it inside the device 1, as described below with reference to Figures 22-24.

In the configuration illustrated in Figure 21, the rotation of the position sensor 130 is blocked by the abutment of a second projecting portion 130a of the position sensor 130 against a deformable abutment surface 135a of the cover 135 of the position sensor 130 (Figures 22 and 23). In addition, as illustrated in Figure 22, a protrusion 172c formed on the rack 172 is in contact with a third projecting portion 130b of the position sensor 130 to absorb the peak force occurring when the tooth 104c comes into abutment with the first projecting portion 130* of the position sensor 130.

The torsion spring 105 of Figures 15 and 16 is still pre-tensioned, but the thrust action exerted by the tooth 104c on the projecting portion 130* is not sufficient to allow the projecting portion 130a to pass the abutment surface 135a due to the deformation of the latter.

As illustrated in Figure 23, the rack 172 further moves as a result of the further rotation of the worm screw 84 of the first motion transmission member 80, and the protrusion 172c of the rack 172 pushes against a further projecting portion 130c of the position sensor 130, exerting on the latter a thrust action suitable to cause the deformation of the abutment surface 135a and the rotation of the position sensor 130 (configuration immediately following the one illustrated in Figure 23).

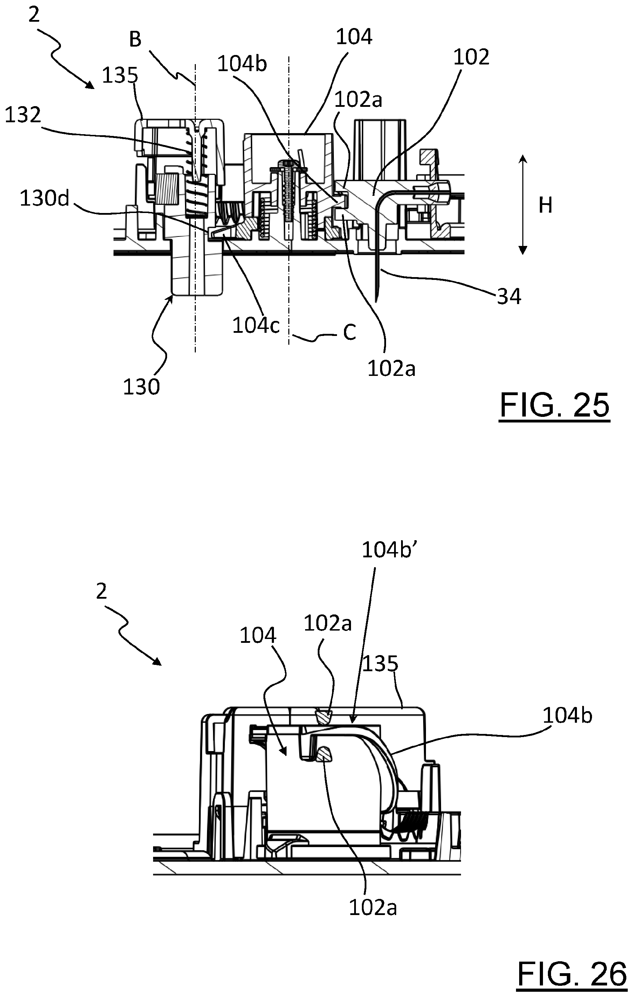

Such a rotation of the position sensor 130 causes the disengagement of the tooth 104c from the first projecting portion 130* of the position sensor 130 (Figure 22), allowing the control member 104 to rotate due to the thrust exerted by the torsion spring 105 (Figure 24). During this rotation, the pair of appendages 103* move following the cam profile 104b from the central portion 104b" to the other end portion 104b', causing the support member 102 to translate along the direction H until the injection needle 34 is pulled out of the patient's skin and retracted into the device 1 (Figure 26).

A solution is provided in the device 1 for allowing the injection needle 34 to be instantly retracted into the device 1 in the event of premature removal of the device 1 by the patient, thereby protecting the patient from exposure of the injection needle 34. This solution is described below.

Figure 25 shows a condition immediately after the device 1 was removed from the patient's skin. In this condition, the injection needle 34 is still outside the device 1 and the position sensor 130 has been pushed by the compression spring 132 towards the outside of the device 1. The appendages 103* are located at the central portion 104b" of the cam profile 104b. During movement of the position sensor 130 outwards from the device 1, a recess 130d formed in the position sensor 130 is arranged at the tooth 104c of the control member 104. The tooth 104c then enters the recess 130d, allowing the control member 104 to rotate due to the thrust exerted by the torsion spring 105. During this rotation, the appendages 103* move following the cam profile 104b from the central portion 104b" to the other end portion 104b', causing the support member 102 to move along the direction H until the injection needle 34 is pulled out of the patient's skin and returned inside the device 1 (Figure 26).

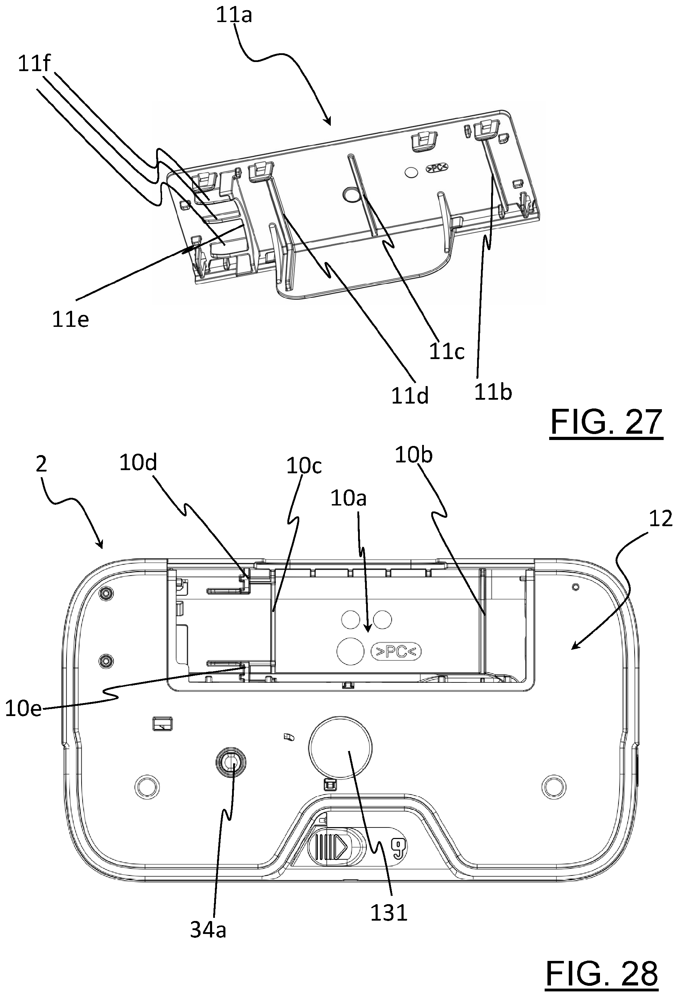

As illustrated in Figures 27-29, a portion 10a of cartridge housing 20 is defined in the housing compartment 10. This portion 10a is closed by a cover 11a.

The Applicant has verified that in the device 1 the cartridge 20, which for example can contain 10 ml of medicament, is subjected to high loads (up to 300 N estimated with a theoretical mathematical model taking into account the higher flow and viscosity values of the medicament) which under critical conditions (blockage of the plunger 25 due to occlusion or end-of-dose situation) can deform the cover 11a.

The Applicant thought to separate the radial centring function of the cartridge 20 in the portion 10a from the axial clamping function of the cartridge 20, so as to be able to control the stresses within the device 1 at the cartridge 20.

In the device 1, the radial centring of the cartridge 20 is achieved by the provision of two ribs 10b, 10c formed in the portion 10a and three ribs 11b, 11c, 11d formed on the cover 11a.

The rib 10b is C-shaped and is arranged in the portion 10a of the housing 10 so as to support the cartridge 20 near its end portion 23a opposite the end portion 23.

The rib 10c is C-shaped and is arranged in the portion 10a of the housing 10 so as to support the cartridge 20 near its end portion 23.

As illustrated in Figure 29, the rib 11d is arranged at the rib 10c of the portion 10a, the rib 11b is arranged at the rib 10b of the portion 10a, and the rib 11c is arranged substantially midway between the ribs 11b and 11d.

The cover 11a also comprises a wall 11e arranged at the end portion 23 of the cartridge 20.

The axial translation of the cartridge 20 is prevented by two walls 10d, 10e protruding from the portion 10a. The more the cartridge 20 is held in the axial position, the more it is able to withstand high loads.

Under high loads, the walls 10d and 10e may deform, leaving the cartridge 20 free to move axially. For this reason, a wall 11e has been provided on the cover 11a. This wall 11e cooperates with the walls 10d and 10e of the portion 10a, acting as an additional axial abutment for the cartridge 20. In this way, a high load (e.g. 300 N) is split between the portion 10a and the cover 11a.

The cover 11a further comprises one or more ribs 11f (three ribs 11f in the example in Figure 27) adapted to reinforce the wall 11e.

The solution described above allows only an axial rotation of the cartridge 20, which, however, does not affect the proper functioning of the device 1. The sequence of operational steps actuated by the device 1 during its use is similar to that described in the above-mentioned patent application WO 2024116017.

The only difference is that in the device 1 the priming step is generally not provided, as the device 1 is typically used to administer the entire quantity of medicament contained in the cartridge 20. However, this priming step could also be carried out in the device 1.

The device 1 can allow the subcutaneous administration of a fixed dose of a medicament in numerous therapeutic areas, such as:
- cardiovascular and renal, e.g. for the following indications: oedema, heart failure, renal disease, membranous nephropathy, focal segmental glomerulosclerosis;
- CNS and neurological, e.g. for the following indications: pain management, Parkinson's disease, restless legs syndrome, multisystem atrophy, neurodegenerative disease, spinal cord injury, acute ischaemic stroke, Alzheimer's disease, dementia, depression, obsessive-compulsive disorder, neuropathic pain post-traumatic stress disorder, spinal muscular atrophy, facioscapulohumeral muscular dystrophy, muscle atrophy, relapsing-remitting multiple sclerosis, primary-progressive multiple sclerosis, secondary-progressive multiple sclerosis, neuromyelitis optica;
- infectious diseases, e.g. for the following indications: infections, human immunodeficiency virus/acquired immunodeficiency syndrome, coronavirus pandemic disease, Streptococcus Pneumoniae;
- cancer, e.g. for the following indications: multiple myeloma, haemolytic anaemia, haematological tumours, chronic lymphatic leukaemia, diffuse large B-cell lymphoma, follicular lymphoma, mantle cell lymphoma, marginal zone lymphoma, T-cell lymphomas, primary mediastinal B-cell lymphoma, aggressive natural killer cell leukaemia, cutaneous T-cell lymphoma, Burkitt's lymphoma, lymphoplasmacytic lymphoma, acute lymphoblastic leukaemia, Hodgkin's lymphoma, non-small cell lung carcinoma (metastatic) squamous cell carcinoma of the head and neck (metastatic), colorectal cancer (metastatic), renal cell carcinoma (metastatic), medullary thyroid carcinoma (metastatic), gastroesophageal cancer (metastatic) malignant mesothelioma (metastatic), breast cancer (metastatic), ovarian cancer (metastatic), melanoma (metastatic), transitional cell carcinoma (metastatic), hepatocellular carcinoma (metastatic), stomach cancer (metastatic), cancer of the gastro-oesophageal junction (metastatic), squamous cell carcinoma of the oesophagus (metastatic), oesophageal cancer (metastatic), squamous cell carcinoma of the skin (metastatic), small cell lung carcinoma (metastatic) nasopharyngeal cancer (metastatic), solid tumours, triple negative breast cancer (metastatic), endometrial cancer (metastatic), osteosarcoma (metastatic), Ewing's sarcoma (metastatic), liposarcoma (metastatic), sarcoma (metastatic) cervical cancer (metastatic), squamous cell carcinoma of the cervix (metastatic), soft tissue sarcoma (metastatic), basal cell carcinoma (metastatic), chordoma, bladder cancer (metastatic), head and neck cancer (metastatic), anaplastic large cell lymphoma, B-cell lymphomas, Merkel cell carcinoma (metastatic), squamous cell carcinoma of the vulva (metastatic), squamous cell carcinoma of the anus (metastatic), penile squamous cell carcinoma (metastatic), neuroendocrine cancer (metastatic), castration-resistant prostate cancer (metastatic), gastrointestinal stromal tumour (metastatic) glioblastoma multiforme, myelodysplastic syndromes, chronic myelomonocytic leukaemia, acute myeloid leukaemia, pancreatic cancer (metastatic), pancreatic ductal adenocarcinoma (metastatic), hepatocellular carcinoma;
- metabolic and endocrine, e.g. for the following indications: diabetes, hypoglycaemia, type 1 diabetes;
- autoimmune and inflammatory conditions, e.g. for the following indications: Sjogren's syndrome, rheumatoid arthritis, systemic lupus erythematosus, cutaneous lupus erythematosus, dermatomyositis, Pemphigus Vulgaris, hidradenitis suppurativa, psoriasis, ulcerative colitis, Crohn's disease, asthma;
- haematology, e.g. for the following indications: neutropenia, cancer/chemotherapy-induced neutropenia, sickle cell anaemia;
- skin disorders, e.g. for the following indications: skin disorders, hidradenitis suppurativa, psoriasis.

Medicaments that are used in the device 1 when this device is used in the above-mentioned therapeutic areas include, for example, the following pharmacological molecules: ketamine, foscarbidopa, foslevodopa, carbidopa hydrate, levodopa, isatuximab-irfc, glucagone, rapastinel, rilpivirina, bafisontamab, dazodalibep, amivantamab-vmjw, mecapegfilgrastim, nisevokitug, pegfilgrastim, prasinezumab, odronextamab, semorinemab, telisotuzumab vedotin, budigalimab, elezanumab, lutikizumab, tarperprumig, Gefurulimab, cobolimab, encelimab, Sabestomig, ociperlimab, litifilimab, ocrelizumab, ezabenlimab, miptenalimab, xentuzumab, Bempikibart, Letolizumab, nivolumab, relatlimab, ulocuplumab, ipilimumab, Rilvegostomig, sovipostobart, mosunetuzumab, ieramilimab, amivantamab, posdinemab, talquetamab, vixarelimab, suvratoxumab, Camoteskimab, Volrustomig, oleclumab, nipocalimab, guselkumab, traxivitug, sabatolimab, spartalizumab, toripalimab-tpzi, orilanolimab, Duvakitug, rozanolixizumab, zampilimab, cabotegravir, plamotamab, isatuximab, toripalimab.

Obviously, a person skilled in the art, in order to meet specific requirements, may make modifications to the present invention, all of which are within the scope of protection defined by the following claims.

## Claims

1. Device (1) for subcutaneous delivery of a medicament, comprising:
- a rotor (17) selectively controllable in rotation in a first rotation direction (R1) and in a second rotation direction (R2) opposite the first rotation direction (R1);
- a motion transmission member (70) coupled to the rotor (17) and configured to transfer motion from the rotor (17) to a first motion transmission member (80) when the rotor (17) rotates in said first rotation direction (R1) and from the rotor (17) to a second motion transmission member (85) when the rotor (17) rotates in said second rotation direction (R2), wherein said first motion transmission member (80) and said second motion transmission member (85) are arranged on opposite sides with respect to the motion transmission member (70);
**characterised in that** the motion transmission mechanism (70) comprises a service shaft (74) selectively movable towards the first motion transmission mechanism (80) and the second motion transmission mechanism (85) so as to:
- be coupled to the first motion transmission member (80) and decoupled from the second motion transmission member (85) when the rotor (17) rotates in said first rotation direction (R1);
- be coupled to the second motion transmission member (85) and decoupled from the first motion transmission member (80) when the rotor (17) rotates in said second rotation direction (R2).

2. Device (1) according to claim 1, wherein the motion transmission mechanism (70) further comprises a worm screw (72) solidly associated with the rotor (17) and a toothed wheel (75) solidly associated with the service shaft (74) and engaged with the worm screw (72).

3. Device (1) according to claim 2, wherein the worm screw (72) comprises a first helical toothing (72a) and the toothed wheel (75) comprises a second helical toothing (75a) conjugated to the first helical toothing (72a).

4. Device (1) according to claim 3, wherein the second helical toothing (75a) has an angle of inclination, with respect to a plane containing the axis of rotation (A) of the toothed wheel (75), comprised between 5° and 30°.

5. Device (1) according to any one of the previous claims, wherein the service shaft (74) comprises a first end portion (74a) configured to be rotationally coupled to the first motion transmission member (80) and a second end portion (74b) opposite the first end portion (74a) and configured to be rotationally coupled to the second motion transmission member (85).

6. Device (1) according to claim 5, wherein said first motion transmission member (80) comprises a first axial hole (82) configured to house said first end portion (74a) when said first end portion (74a) is rotationally coupled to the first motion transmission member (80) and the second motion transmission member (85) comprises a second axial hole (87) configured to house said second end portion (74b) when said second end portion (74b) is rotationally coupled to the second motion transmission member (85).

7. Device (1) according to claim 5 or 6, wherein the first motion transmission member (80) comprises a first abutment element (83) and the second motion transmission member (85) comprises a second abutment element (88), and wherein said first end portion (74a) comprises a first thrust element (78a) configured to cooperate in abutment with said first abutment element (83) when said first end portion (74a) is rotationally coupled to the first motion transmission member (80) and said second end portion (74b) comprises a second thrust element (78b) configured to cooperate in abutment with said second abutment element (88) when said second end portion (74b) is rotationally coupled to the second motion transmission member (85).

8. Device (1) according to any one of the previous claims, wherein the motion transmission mechanism (70) is housed within a pre-assembled unit (90) and wherein the first motion transmission member (80) and the second motion transmission member (85) are supported by said pre-assembled unit (90).

9. Device (1) according to claim 8 when depending on any one of claims 5 to 7, wherein the service shaft (74) comprises a pair of annular grooves (760, 770) arranged between said first end portion (74a) and said second end portion (74b), and wherein the pre-assembled unit (90) comprises at least one elastically deformable coupling element (910) arranged in proximity to said pair of annular grooves (760, 770), said at least one coupling element (910) comprising a free end portion (921) housed in a first annular groove (760) of said pair of annular grooves (760, 770) when the service shaft (74) is rotationally coupled to the first motion transmission member (80) and in a second annular groove (770) of said pair of annular grooves (760, 770) when the service shaft (74) is rotationally coupled to the second motion transmission member (85).

10. Device (1) according to claim 8 or 9 when depending on claim 2, wherein the first motion transmission member (80) and the second motion transmission member (85) are in axial abutment against respective inner walls (92a, 92b; 94a, 94b) of said pre-assembled unit (90) and wherein the worm screw (72) is in abutment against a further inner wall of said pre-assembled unit (90).

11. Device (1) according to any one of the previous claims, wherein said rotor (17) is a driven rotor (17) selectively controllable in rotation by a driving rotor (7) due to a magnetic coupling between said driven rotor (17) and said driving rotor (7) and wherein the driving rotor (7) is selectively controllable in rotation in said first rotation direction (R1) and said second rotation direction (R2) by a motor (5).

12. Device (1) according to claim 11, wherein each of the driving rotor (7) and driven rotor (17) comprises at least one first permanent magnet (7a, 17a) having a first polarity and at least one second permanent magnet (7b, 17b) having a second polarity opposite the first polarity.

13. Device (1) according to claim 12, wherein the driving rotor (7) is arranged above the driven rotor (17) and wherein each of the driving rotor (7) and driven rotor (17) comprises at least one pair of first permanent magnets (7a, 17a) having said first polarity and at least one pair of second permanent magnets (7b, 17b) having said second polarity, each magnet of said at least one pair of first permanent magnets (7a) of the driving rotor (7) and of said at least one pair of second permanent magnets (7b) of the driving rotor (7) being arranged above a respective area (17h) defined between a respective magnet of said at least one pair of first permanent magnets (17a) of the driven rotor (17) and a respective magnet of said at least one pair of second permanent magnets (17b) of the driven rotor (17).

14. Device (1) according to claim 13, wherein in each of said driving rotor (7) and driven rotor (17) the magnets of said at least one pair of first permanent magnets (7a, 17a) and of said at least one pair of second permanent magnets (7b, 17b) are circumferentially arranged one after the other, the magnets of said at least one pair of first permanent magnets (7a, 17a) being circumferentially interposed between the magnets of said at least one pair of second permanent magnets (7b, 17b).

15. Device (1) according to any one of claims 11 to 14, wherein at least one between the driving rotor (7) and the driven rotor (17) comprises a ferromagnetic ring (7f, 17f) arranged on the opposite side with respect to the other of the driving rotor (7) and the driven rotor (17).
